# EUROPEAN PATENT APPLICATION

(11) **EP 3 266 454 A1**
(43) Date of publication of application: **10.01.2018**
(21) Application number: 16178441.8
(22) Date of filing: 07.07.2016
(51) Int. Cl.: A61K 31/427, A61K 31/5377, A61K 31/00, A61K 45/06, A61K 31/133, A61K 31/4409, A61K 31/496, A61K 31/4965, A61P 31/04, A61P 31/06, A61P 31/08

(54) **ACC INHIBITORS FOR USE IN TREATING MYCOBACTERIAL DISEASES**

(71) Applicant: Forschungszentrum Borstel Leibniz-Zentrum für Medizin und Biowissenschaften, 23845 Borstel (DE)
(72) Inventor: Reiling, Norbert, 22926 Ahrensburg (DE); Brandenburg, Julius, 22765 Hamburg (DE)
(74) Representative: Kröncke, Rolf

(57) **Abstract**

The invention relates to acetyl-CoA-carboxylase (ACC) inhibitors and to pharmaceutical compositions containing such compounds, for use in treating mycobacterial diseases. The invention further relates to a kit of parts comprising a pharmaceutical composition containing such compounds and at least one additional pharmaceutically active compound, selected from antbiotics, antifungals or anti-HIV compounds.

## Description

The invention relates to compounds which are suitable for treating mycobacterial diseases and to pharmaceutical compositions containing such compounds. Also encompassed are such compounds for use in medicine. The invention further relates to a kit of parts comprising a pharmaceutical composition containing such compounds and at least one additional pharmaceutically active compound.

Mycobacteria are a diverse collection of acid-fast, non-motile, gram-positive bacteria. It comprises several species, which include, *Mycobacterium africanum (M. africanum), M. avium, M. bovis,* M. *bovis-BCG, M. chelonae, M. fortuitum, M. gordonae, M. intracellulare, M. kansasii, M. microti, M. scrofulaceum, M. paratuberculosis,* M. *leprae,* M. *tuberculosis,* and *M. ranae.* Certain of these organisms are the causative agents of disease. Tuberculosis and leprosy (Hansen's disease) are the best known mycobacterial diseases. People may also be infected by any of a group of mycobacterial species collectively called non-tuberculous mycobacteria (NTM). In children, NTM cause lymphadenitis, skin and soft tissue infections, and occasionally also lung disease and disseminated infections. Manifestations can be indistinguishable from tuberculosis on the basis of clinical and radiological findings and tuberculin skin testing. Although over 150 different species of NTM have been described, pulmonary infections are most commonly due to *Mycobacterium avium* complex (MAC), *Mycobacterium kansasii,* and *Mycobacterium abscessus.*

Tuberculosis (TB) is one of the world's most prevalent infectious diseases responsible for the largest fraction of infection-related casualties (before HIV). The disease is caused by bacteria of the *Mycobacterium tuberculosis* complex. Species in this complex include *M. tuberculosis, M. africanum, M. bovis, M. caprae* etc, whereby *M. tuberculosis* is the main causative agent of tuberculosis. Tuberculosis usually affects the lung and represents the most frequent form of TB. Worldwide, TB had an estimated incidence of 9.6 million people with 1.5 million annual deaths in 2014.

Tuberculosis is usually controlled using extended antibiotic therapy. There are six front-line drugs known to be highly effective against *M. tuberculosis* and several second-line drugs including streptomycin as well as third-line drugs, which are used when resistance to one or more of the front-line drugs is detected. The preferred mode of treatment for tuberculosis is the short course chemotherapy in which there are two phases. The first phase consists of a daily regimen with isoniazid (300 mg), rifampicin (600 mg), pyrazinamide (3 g) and ethambutol (1.5 g) for two months. The second phase or the continuation phase consists of a daily regimen with isoniazid and rifampicin for the next four months. The vast majority of individuals infected with drug susceptible *M. tuberculosis* strains can be effectively cured when medicines are provided and taken properly. However, inappropriate or incorrect use of anti-mycobacterial drugs, or use of ineffective formulations of drugs (e.g. use of single drugs, poor quality medicines or bad storage conditions), and premature treatment interruption can cause drug resistance of the bacteria, which can then be transmitted, especially in crowded settings such as prisons and hospitals. According to WHO estimates, 0.48 million people were infected with strains resistant to isoniazid and rifampicin (MDR-TB; multidrug-resistant tuberculosis) in 2014 leading to 0.19 million deaths (WHO, *Global tuberculosis report.* 2014). About 9% of MDR-TB patients were infected with an almost untreatable extensively drug-resistant (XDR-TB) variant.

*Mycobacterium tuberculosis* is transmitted by aerosol and is initially taken up by alveolar lung macrophages, which phagocytose but don't kill the bacterium. Most infected individuals can control the disease for a long time. Estimates believe that one-third of the world's population is latently infected (LTBI; latent tuberculosis infection). The bacteria adapt and survive in diverse environmental niches *in vivo,* e.g. in solid granulomas, a characteristic feature of latent TB infection. It is presumed that *M. tuberculosis* resides in these regions in a slow growing or non-replicating, phenotypically drug resistant dormant-like state, due to limited availability and supply of oxygen and nutrients (Gengenbacher, M. and S.H. Kaufmann, Mycobacterium tuberculosis: success through dormancy. FEMS Microbiol Rev, 2012. 36(3): p. 514-32).

During infection it has been shown that *M. tuberculosis* accumulates triacylglycerols (TAGs) within intracellular inclusion bodies (Garton, N.J., et al., Intracellular lipophilic inclusions of mycobacteria in vitro and in sputum. Microbiology, 2002. 148(Pt 10): p. 2951-8). The hydrolysis of TAGs to free fatty acids is an essential prerequisite for *M. tuberculosis* growth and survival requiring diverse lipases and hydrolases.

Moreover, it is believed that these fatty acids are crucial for bacteria to enter and maintain the dormant state by the production of foamy lung macrophages during latent infection. Specifically, targeting the bacterial lipid metabolism could represent a viable strategy to limit the growth of *M. tuberculosis* and open a new opportunity to shorten the long TB therapy (Warner DF, Mizrahi V. Shortening treatment for tuberculosis--to basics. N Engl J Med, 2014. 371 (17):1642-3), e.g. when given simultaneously with known first and second line antibiotics.

Acetyl-CoA-carboxylase (ACC) is a biotin-dependent enzyme that catalyzes the irreversible carboxylation of acetyl-CoA to produce malonyl-CoA through its two catalytic activities, namely, biotin carboxylase and carboxyl transferase. ACC can be found as a multi-subunit enzyme in most prokaryotes and in the chloroplasts of most plants and algae, whereas it is a large multidomain enzyme in the endoplasmic reticulum of most eukaryotes. While in prokaryotes and in the chloroplasts of most plants and algae only one type of ACC is present, in eukaryotes like mammals including human two different genes encoding ACCs can be determined. In the human genome, the genes for the two different ACCs are ACACA and ACACB. The main structural difference of the two isoforms present in mammals is in the extended ACC2 N-terminus containing a mitochondria targeting sequence. That is, while the ACC2, acetyl-CoA-carboxylase-2, can be determined in mitochondria, the acetyl-CoA-carboxylase-1 (ACC1) is localized in the cytoplasm of the eukaryotes.

The ACC enzymes play pivotal roles in energy homeostasis, not only through direct participation in fatty acid synthesis, but also through regulation of fatty acid b-oxidation. Malonyl-CoA is a key metabolite that serves as a precursor for de novo fatty acid synthesis. However malonyl-CoA also serves as a sensor to gauge the rate of mitochondrial fatty acid b-oxidation through an allosteric inhibition of carnitine palmitoyl-CoA transferase (CPT-1). The carnitine palmitoyltransferase system is an essential and rate limiting step in the beta-oxidation of long chain fatty acids. This transfer system is necessary because, while fatty acids are activated (in the form of a thioester linkage to coenzyme A) on the outer mitochondrial membrane, the activated fatty acids must be oxidized within the mitochondrial matrix. Long chain fatty acids such as palmitoyl-CoA, unlike short- and medium-chain fatty acids, cannot freely diffuse through the mitochondrial inner membrane, and require a shuttle system to be transported to the mitochondrial matrix.

With respect to the two different isoforms of ACC present in mammals, ACC1 and ACC2, it is submitted that in view of the differences in tissue distribution, ACC1 may maintain regulation of fatty acid synthesis, while ACC2 may mainly regulate fatty acid oxidation.

Regulation of both enzymes is on transcriptional level but is also based on the sensitivity to nutritional status and a feed forward loop. At the moment, it is submitted that ACC presents clinical possibilities in the development of new therapies for diabetes, obesity and other manifestations of metabolic syndromes. In addition, it is submitted that due to the differences between the bacterial ACC and mammal ACC, antibiotics specific to the bacterial ACC may be developed.

Glund et al, Diabetologia, 2012, 55: p2044-53 disclose that inhibition of ACC2enhances skeletal muscle fatty acid oxidation and improves whole-body glucose homeostasis in *db*/*db* mice. A review of ACC inhibitors is given by Corbett, 2008, Expert Opinion on Therapeutic Patents, 19(7): 943-56. Fullerton et al, Nature Medicine, 2013, 19 (12): 1649-54 describe that single phosphorylation sites in ACC1 and ACC2 regulate lipid homeostasis and the insulin-sensitizing effects of metformin a compound known as an active agent in treating type 2 diabetes. Metformin was also recently described as a compound reducing the intracellular growth of *M, tuberculosis,* see Singhal A., et al, Science Translational Medicine, 2014, 6 (263), 236ra159.

There is an urgent medical need to identify new means and methods with significant therapeutic activity against mycobacterial diseases in general and in particular against single-or multiple-drug resistant strains of *M. tuberculosis* and with pharmacokinetic properties that permit reduced dosing resulting also in the reduction of side effects which will in turn encourage better compliance.

The underlying problem of the present invention is the provision of new chemical agents having significant therapeutic activity against mycobacterial diseases, allowing inhibiting the extracellular and intracellular growth of mycobacteria.

The inventors of the present invention have conducted intensive studies and found surprisingly that inhibitors of the acetyl-CoA-transferase (ACC), in particular, inhibitors effective against the ACC2 represents a suitable agent allowing decreasing the replication rate of *M. tuberculosis* in the primary host, namely, macrophages while not negatively influencing the macrophages, e.g. killing the same.

It has been found that the compounds, namely, the inhibitors of ACC inhibit the growth of *M. tuberculosis,* in particular the intracellular growth of *M. tuberculosis* in human macrophage host cells, while not affecting the viability of the host cells accordingly.

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

In order that the present invention may be more readily understood, certain terms are first defined. Additional definitions are set forth throughout the description.

As used herein and throughout the entire description, the term "alkyl" refers to a monoradical of a saturated straight or branched hydrocarbon. Preferably, the alkyl group comprises from 1 to 12 (such as 1 to 10) carbon atoms, i.e., 1, 2, 3, 4, 5, 6, 7, 8, 9, 10, 11, or 12 carbon atoms (such as 1, 2, 3, 4, 5, 6, 7, 8, 9, or 10 carbon atoms), more preferably 1 to 8 carbon atoms, such as 1 to 6 or 1 to 4 carbon atoms. Exemplary alkyl groups include methyl, ethyl, propyl, iso-propyl, butyl, iso-butyl, tert-butyl, n-pentyl, iso-pentyl, sec-pentyl, neo-pentyl, 1,2-dimethyl-propyl, iso-amyl, n-hexyl, iso-hexyl, sec-hexyl, n-heptyl, iso-heptyl, n-octyl, 2-ethyl-hexyl, n-nonyl, n-decyl, n-undecyl, n-dodecyl, and the like. In some embodiments the alkyl chain is a linear. In some embodiments the alkyl chain is branched. In some embodiments the alkyl chain is substituted. In some embodiment the alkyl chain is unsubstituted. In some embodiments the alkyl chain is linear and substituted or unsubstituted. In some embodiments the alkyl chain is branched and substituted or unsubstituted.

As used herein and throughout the entire description, the term "alkenyl" refers to a monoradical of an unsaturated straight or branched hydrocarbon having at least one carbon-carbon double bond. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 to 4, i.e., 1, 2, 3, or 4, carbon-carbon double bonds. Preferably, the alkenyl group comprises from 2 to 10 carbon atoms, i.e., 2, 3, 4, 5, 6, 7, 8, 9, or 10 carbon atoms, more preferably 2 to 8 carbon atoms, such as 2 to 6 carbon atoms or 2 to 4 carbon atoms. Thus, in a preferred embodiment, the alkenyl group comprises from 2 to 10 carbon atoms and 1, 2, 3, 4, or 5 carbon-carbon double bonds, more preferably it comprises 2 to 8 carbon atoms and 1, 2, 3, or 4 carbon-carbon double bonds, such as 2 to 6 carbon atoms and 1, 2, or 3 carbon-carbon double bonds or 2 to 4 carbon atoms and 1 or 2 carbon-carbon double bonds. The carbon-carbon double bond(s) may be in cis (Z) or trans (E) configuration. Exemplary alkenyl groups include vinyl, 1-propenyl, 2-propenyl (i.e., allyl), 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, and the like. If an alkenyl group is attached to a nitrogen atom, the double bond cannot be alpha to the nitrogen atom. In some embodiments the alkenyl chain is a linear. In some embodiments the alkenyl chain is branched. In some embodiments the alkenyl chain is substituted. In some embodiment the alkenyl chain is unsubstituted. In some embodiments the alkenyl chain is linear and substituted or unsubstituted. In some embodiments the alkenyl chain is branched and substituted or unsubstituted.

As used herein and throughout the entire description, the term "alkynyl" refers to a monoradical of an unsaturated straight or branched hydrocarbon having at least one carbon-carbon triple bond. Generally, the maximal number of carbon-carbon triple bonds in the alkynyl group can be equal to the integer which is calculated by dividing the number of carbon atoms in the alkynyl group by 2 and, if the number of carbon atoms in the alkynyl group is uneven, rounding the result of the division down to the next integer. For example, for an alkynyl group having 9 carbon atoms, the maximum number of carbon-carbon triple bonds is 4. Preferably, the alkynyl group has 1 to 4, i.e., 1, 2, 3, or 4, more preferably 1 or 2 carbon-carbon triple bonds. Preferably, the alkynyl group comprises from 2 to 10 carbon atoms, i.e., 2, 3, 4, 5, 6, 7, 8, 9, or 10 carbon atoms, more preferably 2 to 8 carbon atoms, such as 2 to 6 carbon atoms or 2 to 4 carbon atoms. Thus, in a preferred embodiment, the alkynyl group comprises from 2 to 10 carbon atoms and 1, 2, 3, 4, or 5 (preferably 1, 2, or 3) carbon-carbon triple bonds, more preferably it comprises 2 to 8 carbon atoms and 1, 2, 3, or 4 (preferably 1 or 2) carbon-carbon triple bonds, such as 2 to 6 carbon atoms and 1, 2 or 3 carbon-carbon triple bonds or 2 to 4 carbon atoms and 1 or 2 carbon-carbon triple bonds. Exemplary alkynyl groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 2-heptynyl, 3-heptynyl, 4-heptynyl, 5-heptynyl, 6-heptynyl, 1-octynyl, 2-octynyl, 3-octynyl, 4-octynyl, 5-octynyl, 6-octynyl, 7-octynyl, 1-nonylyl, 2-nonynyl, 3-nonynyl, 4-nonynyl, 5-nonynyl, 6-nonynyl, 7-nonynyl, 8-nonynyl, 1-decynyl, 2-decynyl, 3-decynyl, 4-decynyl, 5-decynyl, 6-decynyl, 7-decynyl, 8-decynyl, 9-decynyl, and the like. If an alkynyl group is attached to a nitrogen atom, the triple bond cannot be alpha to the nitrogen atom. In some embodiments the alkynyl chain is a linear. In some embodiments the alkynyl chain is branched. In some embodiments the alkynyl chain is substituted. In some embodiment the alkynyl chain is unsubstituted. In some embodiments the alkynyl chain is linear and substituted or unsubstituted. In some embodiments the alkynyl chain is branched and substituted or unsubstituted.

As used herein and throughout the entire description, the term "aryl" or "aromatic ring" refers to a monoradical of an aromatic cyclic hydrocarbon. Preferably, the aryl group contains 3 to 14 (e.g., 5 to 10, such as 5, 6, or 10) carbon atoms, more preferably 6 to 10 carbon atoms, which can be arranged in one ring (e.g., phenyl) or two or more condensed rings (e.g., naphthyl). Exemplary aryl groups include cyclopropenylium, cyclopentadienyl, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are phenyl and naphthyl. In some embodiments the aryl is unsubstituted. In some embodiments the aryl is substituted.

As used herein and throughout the entire description, the term "heteroaryl" or "heteroaromatic ring" means an aryl group as defined above in which one or more carbon atoms in the aryl group are replaced by heteroatoms of O, S, or N. Preferably, the heteroaryl group contains 3 to 10 carbon atoms. Preferably, heteroaryl refers to a five or six-membered aromatic monocyclic ring wherein 1, 2, or 3 carbon atoms are replaced by the same or different heteroatoms of O, N, or S. Alternatively, it means an aromatic bicyclic or tricyclic ring system wherein 1, 2, 3, 4, or 5 carbon atoms are replaced with the same or different heteroatoms of O, N, or S. Preferably, in each ring of the heteroaryl group the maximum number of O atoms is 1, the maximum number of S atoms is 1, and the maximum total number of O and S atoms is 2. Exemplary heteroaryl groups include furanyl, thienyl, oxazolyl, isoxazolyl, oxadiazolyl (1,2,5- and 1,2,3-), pyrrolyl, imidazolyl, pyrazolyl, triazolyl (1,2,3- and 1,2,4-), tetrazolyl, thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), benzofuranyl (1- and 2-), indolyl, isoindolyl, benzothienyl (1- and 2-), 1H-indazolyl, benzimidazolyl, benzoxazolyl, indoxazinyl, benzisoxazolyl, benzothiazolyl, benzisothiazolyl, benzotriazolyl, quinolinyl, isoquinolinyl, benzodiazinyl, quinoxalinyl, quinazolinyl, benzotriazinyl (1,2,3- and 1,2,4-benzotriazinyl), pyridazinyl, phenoxazinyl, thiazolopyridinyl, pyrrolothiazolyl, phenothiazinyl, isobenzofuranyl, chromenyl, xanthenyl, phenoxathiinyl, pyrrolizinyl, indolizinyl, indazolyl, purinyl, quinolizinyl, phthalazinyl, naphthyridinyl (1,5-, 1,6-, 1,7-, 1,8-, and 2,6-), cinnolinyl, pteridinyl, carbazolyl, phenanthridinyl, acridinyl, perimidinyl, phenanthrolinyl (1,7-, 1,8-, 1,10-, 3,8-, and 4,7-), phenazinyl, oxazolopyridinyl, isoxazolopyridinyl, pyrrolooxazolyl, and pyrrolopyrrolyl. Exemplary 5- or 6-memered heteroaryl groups include furanyl, thienyl, oxazolyl, isoxazolyl, oxadiazolyl (1,2,5- and 1,2,3-), pyrrolyl, imidazolyl, pyrazolyl, triazolyl (1,2,3- and 1,2,4-), thiazolyl, isothiazolyl, thiadiazolyl (1,2,3- and 1,2,5-), pyridyl, pyrimidinyl, pyrazinyl, triazinyl (1,2,3-, 1,2,4-, and 1,3,5-), and pyridazinyl. In some embodiments the heteroaryl is unsubstituted. In some embodiments the heteroaryl is substituted.

As used herein and throughout the entire description, the terms "arylalkyl" and "heteroarylalkyl" are meant to include those radicals in which an aryl group and heteroaryl group, respectively, is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like). Preferably the arylalkyl is a substituted or unsubstituted (C₆-C₁₀)aryl(C₁-C₆)alkyl. Preferably the heteroarylalkyl is a substituted or unsubstituted (C₃-C₁₀)heteroaryl(C₁-C₆)alkyl. In some embodiments the alkyl chain is a linear. In some embodiments the alkyl chain is branched. In some embodiments the alkyl chain is substituted. In some embodiments the alkyl chain is unsubstituted. In some embodiments the alkyl chain is linear and substituted or unsubstituted. In some embodiments the alkyl chain is branched and substituted or unsubstituted. In some embodiments the arylalkyl is unsubstituted. In some embodiments the arylalkyl is substituted. In some embodiments the heteroarylalkyl is unsubstituted. In some embodiments the heteroarylalkyl is substituted.

As used herein and throughout the entire description, the term "cycloalkyl" or "cycloaliphatic" represents cyclic non-aromatic versions of "alkyl" and "alkenyl" with preferably 3 to 14 carbon atoms, such as 3 to 10 carbon atoms, i.e., 3, 4, 5, 6, 7, 8, 9, or 10 carbon atoms, more preferably 3 to 8 carbon atoms, even more preferably 3 to 7 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" is also meant to include bicyclic and tricyclic versions thereof. If bicyclic rings are formed it is preferred that the respective rings are connected to each other at two adjacent carbon atoms, however, alternatively the two rings are connected via the same carbon atom, i.e., they form a spiro ring system or they form "bridged" ring systems. Preferred examples of cycloalkyl include C₃-C₈-cycloalkyl, in particular cyclopropyl, cyclobutyl, cyclopentyl, cyclohexyl, cycloheptyl, cyclooctyl, spiro[3,3]heptyl, spiro[3,4]octyl, spiro[4,3]octyl, bicyclo[4.1.0]heptyl, bicyclo[3.2.0]heptyl, bicyclo[2.2.1]heptyl, bicyclo[2.2.2]octyl, bicyclo[5.1.0]octyl, and bicyclo[4.2.0]octyl. In some embodiments the cycloalkyl is unsubstituted. In some embodiments the cycloalkyl is substituted.

As used herein and throughout the entire description, the term "cyclopropylene" means a cyclopropyl group as defined above in which one hydrogen atom has been removed resulting in a diradical. The cyclopropylene may link two atoms or moieties via the same carbon atom (1,1-cyclopropylene, i.e., a geminal diradical) or via two carbon atoms (1,2-cyclopropylene).

As used herein and throughout the entire description, the term "heterocyclyl" or "heterocyclic ring" or "heterocycle" means a cycloalkyl group as defined above in which from 1, 2, 3, or 4 carbon atoms in the cycloalkyl group are replaced by heteroatoms of O, S, or N. Preferably, in each ring of the heterocyclyl group the maximum number of O atoms is 1, the maximum number of S atoms is 1, and the maximum total number of O and S atoms is 2. The term "heterocyclyl" is also meant to encompass partially or completely hydrogenated forms (such as dihydro, tetrahydro or perhydro forms) of the above-mentioned heteroaryl groups. Exemplary heterocyclyl groups include morpholino, isochromanyl, chromanyl, pyrrolidinyl, imidazolidinyl, pyrazolidinyl, piperidinyl, piperazinyl, indolinyl, isoindolinyl, di- and tetrahydrofuranyl, di- and tetrahydrothienyl, di- and tetrahydrooxazolyl, di- and tetrahydroisoxazolyl, di-and tetrahydrooxadiazolyl (1,2,5- and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydrothiazolyl, di- and tetrahydrothiadiazolyl (1,2,3- and 1,2,5-), di-and tetrahydropyridyl, di- and tetrahydropyrimidinyl, di- and tetrahydropyrazinyl, di-and tetrahydrotriazinyl (1,2,3-, 1,2,4-, and 1,3,5-), di- and tetrahydrobenzofuranyl (1-and 2-), di- and tetrahydroindolyl, di- and tetrahydroisoindolyl, di- and tetrahydrobenzothienyl (1- and 2), di- and tetrahydro-1H-indazolyl, di- and tetrahydrobenzimidazolyl, di- and tetrahydrobenzoxazolyl, di- and tetrahydroindoxazinyl, di- and tetrahydrobenzisoxazolyl, di- and tetrahydrobenzothiazolyl, di- and tetrahydrobenzisothiazolyl, di- and tetrahydrobenzotriazolyl, di- and tetrahydroquinolinyl, di- and tetrahydroisoquinolinyl, di- and tetrahydrobenzodiazinyl, di- and tetrahydroquinoxalinyl, di- and tetrahydroquinazolinyl, di- and tetrahydrobenzotriazinyl (1,2,3- and 1,2,4-), di- and tetrahydropyridazinyl, di- and tetrahydrophenoxazinyl, di- and tetrahydrothiazolopyridinyl (such as 4,5,6-7-tetrahydro[1,3]thiazoio[5,4-c]pyridinyl or 4,5,6-7-tetrahydro[1,3]thiazolo[4,5-c]pyridinyl, e.g., 4,5,6-7-tetrahydro[1,3]thiazolo[5,4-c]pyridin-2-yl or 4,5,6-7-tetrahydro[1,3]thiazolo[4,5-c]pyridin-2-yl), di- and tetrahydropyrrolothiazolyl (such as 5,6-dihydro-4H-pyrrolo[3,4-d][1,3]thiazolyl), di- and tetrahydrophenothiazinyl, di- and tetrahydroisobenzofuranyl, di- and tetrahydrochromenyl, di- and tetrahydroxanthenyl, di- and tetrahydrophenoxathiinyl, di- and tetrahydropyrrolizinyl, di- and tetrahydroindolizinyl, di- and tetrahydroindazolyl, di- and tetrahydropurinyl, di- and tetrahydroquinolizinyl, di- and tetrahydrophthalazinyl, di- and tetrahydronaphthyridinyl (1,5-, 1,6-, 1,7-, 1,8-, and 2,6-), di- and tetrahydrocinnolinyl, di-and tetrahydropteridinyl, di- and tetrahydrocarbazolyl, di- and tetrahydrophenanthridinyl, di- and tetrahydroacridinyl, di- and tetrahydroperimidinyl, di- and tetrahydrophenanthrolinyl (1,7-, 1,8-, 1,10-, 3,8-, and 4,7-), di- and tetrahydrophenazinyl, di-and tetrahydrooxazolopyridinyl, di- and tetrahydroisoxazolopyridinyl, di- and tetrahydropyrrolooxazolyl, and di- and tetrahydropyrrolopyrrolyl. Exemplary 5- or 6-memered heterocyclyl groups include morpholino, pyrrolidinyl, imidazolidinyl, pyrazolidinyl, piperidinyl, piperazinyl, di- and tetrahydrofuranyl, di- and tetrahydrothienyl, di- and tetrahydrooxazolyl, di- and tetrahydroisoxazolyl, di- and tetrahydrooxadiazolyl (1,2,5-and 1,2,3-), dihydropyrrolyl, dihydroimidazolyl, dihydropyrazolyl, di- and tetrahydrotriazolyl (1,2,3- and 1,2,4-), di- and tetrahydrothiazolyl, di- and tetrahydroisothiazolyl, di- and tetrahydrothiadiazolyl (1,2,3- and 1,2,5-), di- and tetrahydropyridyl, di- and tetrahydropyrimidinyl, di- and tetrahydropyrazinyl, di- and tetrahydrotriazinyl (1,2,3-, 1,2,4-, and 1,3,5-), and di- and tetrahydropyridazinyl. In some embodiments the heterocyclyl is unsubstituted. In some embodiments the heterocyclyl is substituted.

As used herein and throughout the entire description, the term "halogen" or "halo" means fluoro, chloro, bromo, or iodo.

As used herein and throughout the entire description, the term "azido" means N₃.

As used herein and throughout the entire description, the term "optionally substituted" or "substituted" indicates that one or more (such as 1 to the maximum number of hydrogen atoms bound to a group, e.g., 1, 2, 3, 4, 5, 6, 7, 8, 9, or up to 10, such as between 1 to 5, 1 to 4, or 1 to 3, or 1 or 2) hydrogen atom(s) may be replaced with a group different from hydrogen such as alkyl (preferably, C₁₋₆ alkyl), alkenyl (preferably, C₂₋₆ alkenyl), alkynyl (preferably, C₂₋₆ alkynyl), aryl (preferably, 3-to 14-membered aryl), heteroaryl (preferably, 3- to 14-membered heteroaryl), cycloalkyl (preferably, 3- to 14-membered cycloalkyl), heterocyclyl (preferably, 3- to 14-membered heterocyclyl), halogen, -CN, azido, -NO₂, -OR⁷¹, -N(R⁷²)(R⁷³), -ON(R⁷²)(R⁷³), -N⁺(-O-)(R⁷²)(R⁷³), -S(O)₀₋₂R⁷¹, -S(O)₀₋₂OR⁷¹, -OS(O)₀₋₂R⁷¹, -OS(0)₀₋₂OR⁷¹, -S(O)₀₋₂N(R⁷²)(R⁷³), -OS(O)₀₋₂N(R⁷²)(R⁷³), -N(R⁷¹)S(O)₀₋₂R⁷¹, -NR⁷¹S(O)₀₋₂OR⁷¹, -NR⁷¹S(O)₀₋₂N(R⁷²)(R⁷³), -C(=W¹)R⁷¹, -C(=W¹)W¹R⁷¹, -W¹C(=W¹)R⁷¹, and -W¹C(=W¹)W¹R⁷¹; wherein R⁷¹, R⁷², and R⁷³ are independently selected from the group consisting of -H, C₁₋₆ alkyl, C₂₋₆ alkenyl, C₂₋₆ alkynyl, 3- to 7-membered cycloalkyl, 5- or 6-membered aryl, 5- or 6-membered heteroaryl, and 3- to 7-membered heterocyclyl, wherein each of the alkyl, alkenyl, alkynyl, cycloalkyl, aryl, heteroaryl, and heterocyclyl groups is optionally substituted with one, two or three substituents selected from the group consisting of C₁₋₃ alkyl, halogen, -CF₃, -CN, azido, -NO₂, -OH, -O(C₁₋₃ alkyl), -S(C₁₋₃ alkyl), -NH₂, -NH(C₁₋₃ alkyl), -N(C₁₋₃ alkyl)₂, -NHS(O)₂(C₁₋₃ alkyl), -S(O)₂NH_{2-z}(C₁₋₃ alkyl)_{z}, -C(=O)OH, -C(=O)O(C₁₋₃ alkyl), -C(=O)NH_{2-z}(C₁₋₃ alkyl)z, -NHC(=O)(C₁₋₃ alkyl), -NHC(=NH)NH_{z-2}(C₁₋₃ alkyl)_{z}, and -N(C₁₋₃ alkyl)C(=NH)NH_{2-z}(C₁₋₃ alkyl)_{z}, wherein z is 0, 1, or 2 and C₁₋₃ alkyl is methyl, ethyl, propyl or isopropyl; W¹ is independently selected from O, S, and NR⁸⁴, wherein R⁸⁴ is -H or C₁₋₃ alkyl.

In a first aspect, the present invention relates to an inhibitor of the acetyl-CoA-carboxylase (ACC) for use in the treatment of mycobacterial disease. It has been recognized by the present inventors that therapeutically effective amounts of an inhibitor of the ACC represents a suitable measure for the treatment of mycobacterial disease. In particular, it has been recognized that by influencing the fatty acid synthesis, like the fatty acid oxidation, in the host, it is possible to inhibit the growth, namely, the intracellular replication of mycobacteria in the host cell without negatively affecting the host cell itself.

In an embodiment, the inhibitor used in a therapeutically effective amount is an ACC2 inhibitor for use in the treatment of mycobacterial disease.

In an embodiment, the inhibitor of the ACC for use in the treatment of mycobacterial disease is an inhibitor having a structure according to formula I 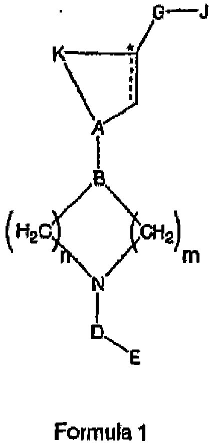 or prodrugs thereof, or pharmaceutically acceptable salts of said inhibitor or of said prodrugs;
wherein
A-B is N-CH or CH-N;
K is (CH₂)r wherein r is 2, 3 or 4;
m and n are each independently 1, 2 or 3 when A-B is N-CH or m and n are each independently 2 or 3 when A-B is CH-N;
the dashed line represents the presence of an optional double bond;
D is carbonyl or sulfonyl;
E is a tricyclic ring consisting of two fused fully unsaturated five to seven membered rings, taken independently, each of said rings optionally having one to four heteroatoms selected independently from oxygen, sulfur and nitrogen, said two fused rings fused to a third partially saturated, fully unsaturated or fully saturated five to seven membered ring, said third ring optionally having one to four heteroatoms selected independently from oxygen, sulfur and nitrogen; or
wherein said E tricyclic ring is optionally mono-, di- or tri-substituted independently on each ring used to form the tricyclic ring with halo, hydroxy, amino, cyano, nitro, oxo, carboxy, (C₁-C₆)alkyl, (C₂-C₆)alkenyi, (C₂-C₈)alkynyl, (C₁-C₆)alkoxy, (C₁-C₄)alkylthio, (C₁-C₆)alkoxycarbonyl, (C₁-C₆)alkylcarbonyl, (C₁-C₆) alkylcarbonylamino, or mono-N-or di-N,N-(C₁-C₆)alkylamino, mono-N- or di-N,N-(C₁-C₆)alkylaminocarbonyl wherein said (C₁-C₆)alkyl, (C₁-C₆)alkoxy and (C₁-C₄)alkylthio substituents are also optionally mono-, di- or tri-substituted independently with chloro, bromo, hydroxy, (C₁-C₆)alkoxy, amino, mono-N- or di-N,N-(C₁-C₆)alkylamino or from one to nine fluorines; and
wherein said E tricyclic ring is optionally mono-substituted with a partially saturated, fully saturated or fully unsaturated three to eight membered ring R¹⁰ optionally having one to four heteroatoms selected independently from oxygen, sulfur and nitrogen or a bicyclic ring R¹¹ consisting of two fused partially saturated, fully saturated or fully unsaturated three to eight membered rings, taken independently, each of said rings optionally having one to four heteroatoms selected independently from oxygen, sulfur and nitrogen, said R¹⁰ and R¹¹ rings optionally additionally bridged and said R¹⁰ and R¹¹ rings optionally linked through a fully saturated, partially unsaturated or fully saturated one to four membered straight or branched carbon chain wherein the carbon(s) may optionally be replaced with one or two heteroatoms selected independently from oxygen, nitrogen and sulfur;
wherein said R¹⁰ or R¹¹ ring is optionally mono-, di or tri-substituted independently with halo, hydroxy, amino, cyano, nitro, oxo, carboxy, (C₁-C₆)alkyl, (C₂-C₆)alkenyl, (C₂-C₆)alkynyl, (C₁-C₆)alkoxy, (C₁-C₄)alkylthio, (C₁-C₆)alkoxycarbonyl, (C₁-C₆)alkylcarbonyl, (C₁-C₆)alkylcarbonylamino, or mono-N- or di-N,N-(C₁-C₆)alkylamino or mono-N- or di-N,N-(C₁-C₆)alkylaminocarbonyl wherein said (C₁-C₆)alkyl and (C₁-C₆)alkoxy substituents are also optionally mono-, di or tri-substituted independently with halo, hydroxy, (C₁-C₆)alkoxy, amino, mono-N-or di-N,N-(C₁-C₆)alkylamino or from one to nine fluorines;
G is carbonyl, sulfonyl or CR¹R⁸,
wherein R⁷ and R⁸ are each independently H, (C₁-C₆)alkyl, (C₂-C₆)alkenyl or (C₂-C₆)alkynyl or a five to seven membered partially saturated, fully saturated or fully unsaturated ring optionally having one heteroatom selected from oxygen, sulfur and nitrogen;
J is OR¹; NR²R³ or CR⁴R⁵R⁶;
wherein R¹, R² and R³ are each independently H, Q, or a (C₁-C₁₀)alkyl, (C₃-C₁₀)alkenyl or (C₃-C₁₀)alkynyl substituent wherein said carbon(s) may optionally be replaced with one or two heteroatoms selected independently from oxygen, nitrogen and sulfur and wherein said sulfur is optionally mono- or di-substituent with oxo, said carbon(s) is optionally mono-substituted with oxo, said nitrogen is optionally disubstituted with oxo, said carbon(s) is optionally mono-, di- or tri-substituted independently with halo, hydroxy, amino, nitro, cyano, carboxy, (C₁-C₄)alkylthio, (C₁-C₆)alkyloxycarbonyl, mono-N- or di-N,N-(C₁-C₆)alkylamino or mono-N- or di-N,N-(C₁-C₆)alkylamino-carbonyl;
and said chain is optionally mono-substituted with Q¹;
wherein Q amd Q1 are each independently a partially saturated, fully saturated or fully unsaturated three to eight membered ring optionally having one to three heteroatoms selected independently from oxygen, sulfur and nitrogen or a bicyclic ring consisting of two fused or spirocyclic partially saturated, fully saturated or fully unsaturated three to six membered rings, taken independently, said bicyclic ring optionally having one to three heteroatoms selected independently form oxygen, sulfur and nitrogen, said mono or bicyclic ring optionally additionally bridged with (C₁-C₃)alkylene wherein said (C₁-C₃)alkylene carbons are optionally replaced with one to two heteroatoms selected independently from oxygen, sulfur and nitrogen;
wherein said Q and Q1 ring are each independently optionally mono-, di-, tri-, or tetra-substituted independently with halo, hydroxy, amino, nitro, cyano, oxo, carboxy, (C₁-C₆)alkyl, (C₂-C₆)alkenyl, (C₂-C₆)alkynyl, (C₁-C₆) alkoxy, (C₁-C₄)alkylthio, (C₁-C₆)alkoxycarbonyl, (C₁-C₆)alkylcarbonyl, (C₁-C₆)alkylcarbonylamino, (C₁-C₆)alkyloxycarbonyl, mono-N- or di-N,N-(C₁-C₆)alkylamino, mono-N- or di-N,N-(C₁-C₆)alkylaminosulfonyl, mono-N- or diN,N-(C₁-C₆)alkylaminocarbonyl, wherein said (C₁-C₆)alkyl substituent is optionally mono-, di or tri-substituted independently with halo, hydroxy, amino, nitro, cyano, oxo, carboxy, (C₁-C₆)alkoxy, (C₁-C₄)alkylthio, (C₁-C₆)alkyloxycarbonyl or mono-N- or di-N,N-(C₁-C₆)alkylamino wherein said (C₁-C₆)alkyl substituent is also optionally substituted with from one to nine fluorines;
or wherein R² and R³ can be taken together with the nitrogen atom to which they are attached to form a partially saturated, fully saturated or fully unsaturated three to eight membered ring optionally having one to three additional heteroatoms selected independently form oxygen, sulfur and nitrogen or a bicyclic ring consisting of two fused, bridged or spirocyclic partially saturated, fully saturated or fully unsaturated three to six membered rings, taken independently, said bicyclic ring optionally having one to three additional heteroatoms selected independently from oxygen, sulfur and nitrogen or a tricyclic ring consisting of three fused, bridged or spirocyclic partially saturated, fully saturated or fully unsaturated three to six membered rings, taken independently, said tricyclic ring optionally having one to three additional heteroatoms selected independently from oxygen, sulfur and nitrogen;
wherein said NR²R³ ring is optionally mono-, di-, tri- or tetra-substituted independently with R¹⁵, halo, hydroxy, amino, nitro, cyano, oxo, carboxy, (C₁-C₆)alkyl, (C₂-C₆)alkenyl, (C₂-C₆)alkynyl, (C₁-C₆)alkoxy, (C₁-C₄)aikylthio, (C₁-C₆)alkylcarbonylamino or mono-N- or di-N,N-(C₁-C₆)alkylamino, wherein said (C₁-C₆)alkyl substituent is optionally mono-, di- or tri-substituted independently with halo, hydroxy, amino, nitro, cyano, oxo, carboxy, (C₁-C₆)alkoxy, (C₁-C₄)alkylthio, (C₁-C₆)alkyloxycarbonyl, mono-N or di-N,N-(C₁-C₆)alkylamino, said (C₁-C₆)alkyl substituent is also optionally substituted with from one to nine fluorines;
wherein R¹⁵ is carbonyl, carbamoyl, sulfonyl or sulfamoyl substituted with H, (C₁-C₆)alkyl, (C₁-C₆)alkyloxy, (C₁-C₆)alkyloxycarbonyl, (C₁-C₆)alkyloxycarbonyl(C₁-C₆)alkyl, mono-N- or di-N,N-(C₁-C₆)alkylamino,
wherein said (C₁-C₆)alkyl substituent is optionally mono-, di or tri-substituted independently with halo, hydroxy, amino, nitro, cyano, oxo, carboxy, (C₁-C₆)aikoxy, (C₁-C₄)alkylthio, (C₁-C₆)alkyloxycarbonyl, (C₁-C₆)alkylcarbonyloxy, mono-N- or di-N,N-(C₁-C₆)alkylamino or the R¹⁵ carbonyl, carbamoyl, sulfonyl or sulfamoyl linked substituent is a partially saturated, fully saturated or fully unsaturated three to eight membered ring optionally linked through (C₁-C₆)alkyl and optionally having one to three heteroatoms selected independently from oxygen, sulfur and nitrogen wherein said ring is optionally mono-, di- or tri-substituted with halo, hydroxy, amino, nitro, cyano, oxo, carboxy, (C₁-C₆)alkyl, (C₂-C₆)alkenyl, (C₂-C₆)alkynyl, (C₁-C₄)alkylthio, (C₁-C₆)alkoxy, (C₁-C₆)aikylcarbonylamino, mono-N- or di-N,N-(C₁-C₆)alkylamino; wherein said NR²R³ ring is optionally substituted with a partially saturated, fully saturated or fully unsaturated three to eight membered ring optionally having one to three heteroatoms selected independently from oxygen, sulfur and nitrogen or a bicyclic ring consisting of two fused partially saturated, fully saturated o fully unsaturated three to six membered rings, taken independently, said bicyclic ring optionally having one to three heteroatoms selected independently form oxygen, sulfur and nitrogen, said mono or bicyclic ring optionally additionally bridged said ring optionally having one to three heteroatoms selected independently from oxygen, sulfur and nitrogen, wherein said (C₁-C₆)alkyl and said ring are optionally mono-, di- or tri-substituted with halo, hydroxy, amino, nitro, cyano, oxo, carboxy, (C₂-C₆)alkenyl, (C₃-C₆)alkynyl, (C₁-C₆)alkylcarbonylamino, hydroxy, (C₁-C₆)alkoxy, (C₁-C₄)alkylthio, (C₁-C₆)alkoxy, mono-N- or di-N,N-(C₁-C₆)alkylamino;
wherein R⁴, R⁵, R⁶ are independently H, halo, hydroxy, (C₁-C₆)alkyl or R⁴ and R⁵ are taken together to form a partially saturated, fully saturated or fully unsaturated three to eight membered ring, said ring optionally having one to three heteroatoms selected independently from oxygen, sulfur and nitrogen, wherein said (C₁-C₆)alkyl and said ring are optionally mono-, di- or tri-substituted with halo, hydroxy, amino, nitro, cyano, oxo, carboxy, (C₂-C₆)alkenyl, (C₃-C₆)alkynyl, (C₁-C₆)alkylcarbonylamino, hydroxy, (C₁-C₆)alkoxy, (C₁-C₄)alkylthio, (C₁-C₆)alkoxy, mono-N- or di-N,N-(C₁-C₆)alkylamino,
or a pharmaceutically acceptable salt, solvate or hydrate thereof.

In a further embodiment, the inhibitor is an inhibitor of the ACC for use in the treatment of mycobacterial disease wherein the inhibitor is an inhibitor having a structure according to formula II 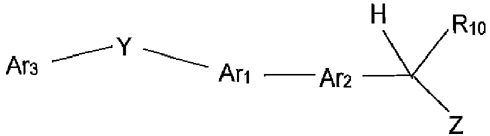
wherein R₁₀ is selected from the group consisting of hydrogen, cycloalkyl, alkyl and haloalkyl;
Y is selected form the group consisting of -(CR₄ₐR_{4b})ₚ, -C(O)-, -O-, -N(H)-, -N(alkyl)-and -S-; wherein
p is 1, 2 or 3;
each of R₄ₐ, R_{4b}, at each occurrence, is independently selected from the group consisting of hydrogen, alkyl, hydroxyalkyl, and haloalkyl when p is 1, 2 or 3; alternatively, R₄ₐ and R_{4b} together with the carbon to which they are attached form a monocyclic cycloalkyl or heterocycle ring when p is 1;
Ar₃ is 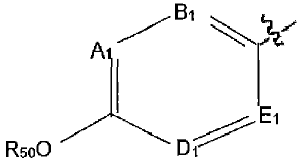
   A₁, B₁, E₁, and D₁ are -C(R)-; or one of A₁, B₁, E₁ and D₁ is N and the others are-C(R)-;
   wherein R is selected from the group consisting of hydrogen, -I, -Br, -Cl, and -F;
   Ar₁ is selected from the group consisting of phenyl, pyridinyl, thienyl, furanyl, thiazolyl, and 1, 3, 4-thiadiazolyl; each of which is independently unsubstituted or substituted with one substituent selected form the group consisting of -I, -Br, -Cl, and -F;
   Ar₂ is selected from the group consisting of thienyl, thiazolyl, isoxazolyl, 1,2,4-thiadiazolyl, and 1,2,4-oxadiazolyl; each of which is independently unsubstituted or substituted with one C₁-C₆ alkyl;
   R₁₀ is selected form the group consisting of C₁-C₆ alkyl and haloalkyl;
   Z is selected form the group consisting of -OR^{9a} and -NR₆₀R_{9b}; wherein R₉ₐ is-S(O)₂(C₁-C₆ alkyl), R₆₀ is hydrogen, and R_{9b} is selected from the group consisting of hydrogen, -C(O)NH₂, -C(O)N(H)(C₁-C₆ alkyl), -C(O)O(C₁-C₆ alkyl), -S(O)₂(C₁-C₆ alkyl), -CH₂-C(O)O(C₁-C₆ alkyl) and -C(O)R₂₀ wherein R₂₀ is C₁-C₆ alkyl or unsubstituted C₁-C₆ cycloalkyl;
   Y is -O-; and
   R₅₀ is selected from the group consisting of C₁-C₆ alkyl, -R₈₀ and -(C₁-C₆ alkylenyl)-R₈₀; wherein R₈₀ at each occurrence is an unsubstituted ring selected from the group consisting of phenyl, cyclopropyl, cyclopentyl, cyclohexyl, tetrahydrofuranyl and tetrahydropyranyl,
   or a pharmaceutically acceptable salt, solvate or hydrate thereof.

In another aspect, the present invention relates to a pharmaceutical composition for use in the treatment of a mycobacterial disease wherein said composition comprises an inhibitor of the ACC, like an inhibitor of the ACC2. In an embodiment, the pharmaceutical composition is a composition comprising a compound having a structure according to formula I as defined herein or of formula II as defined herein.

In the following embodiments the compound for use in the treatment of a mycobacterial disease as disclosed herein above, the compound for use in medicine as disclosed herein above and the compound of the composition for use in the treatment of a mycobacterial disease as disclosed herein above are further defined.

In some embodiments, the compounds for use or the compounds of the pharmaceutical composition for use having a structure according to formula I as disclosed herein are compounds of formula I wherein E is a tricyclic ring, linked through the middle ring, consisting of two fused fully unsaturated six membered rings, taken independently each of said rings optionally having a nitrogen heteroatom, said two fused rings fused to a third partially saturated or fully unsaturated six membered ring, said third ring optionally having one nitrogen heteroatom;
wherein said E ring is optionally mono-, di- or tri-substituted independently on each ring used to form the tri-cyclic ring with halo, hydroxy, (C₁-C₆)alkyl, (C₁-C₆)alkoxy, (C₁-C₄)alkylthio, or mono-N- or diN, N-(C₁-C₆)alkylamino wherein said (C₁-C₆)alkyl and (C₁-C₆)alkoxy substituents are also optionally mono-, di- or tri-substituted independently with halo, hydroxy or from one to nine fluorines; and J is NR²R³,
wherein R² and R³ can be taken together with the nitrogen atom to which they are attached to form a partially saturated or fully saturated five to six membered ring optionally having one additional heteroatom selected independently from oxygen and nitrogen;
wherein said NR²R³ ring is optionally mono-, di-, tri- or tetra-substituted independently with halo, hydroxy, amino, oxo, (C₁-C₆)alkyl, (C₁-C₆)alkoxy wherein said (C₁-C₆)alkyl substituent is optionally mono-, di- or tri-substituted independently with chloro, hydroxy, oxo, (C₁-C₆)alkoxy and said (C₁-C₆)alkyl substituent is also optionally substituted with from one to nine fluorines;
or wherein R² and R³ are each independently H, Q, or (C₁-C₆)alkyl,
wherein said (C₁-C₆)alkyl is optionally mono-, di- or tri-substituted independently with halo, hydroxy, (C₁-C₄)alkylthio, (C₁-C₆)alkyloxycarbonyl, or mono-N- or di-N,N-(C₁-C₆)alkylamino or Q¹;
wherein Q and Q¹ are each independently partially saturated, fully saturated or fully unsaturated three to seven membered ring optionally having one heteroatom selected independently from oxygen and nitrogen;
wherein said Q and Q¹ ring are each independently optionally mono-, di- or tri-substituted independently with halo, hydroxy, oxo, (C₁-C₆)alkyl or (C₁-C₆)alkoxy wherein said (C₁-C₆)alkyl substituent is also optionally substituted with from one to nine fluorines or a pharmaceutically acceptable salt thereof.

In an embodiment of said compound of the structure of formula I is the compound

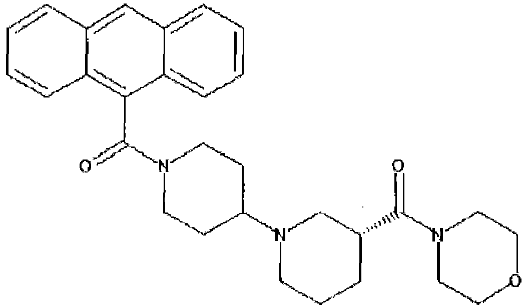

### [(3R)-1'-(9-amthracenylcarbonyl)[1,4'-bipiperidin]-3-yl]-4-morpholinyl-methanone

In other embodiments, the compounds for use or the compound of the pharmaceutical composition for use having a structure according to formula II as disclosed herein are compounds of formula II wherein Ar₃ is 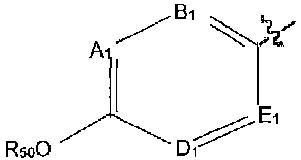
A₁, B₁, E₁, and D₁ are -C(R)-; or one of A₁, B₁, E₁ and D₁ is N and the others are -C(R)-;
wherein R is selected from the group consisting of hydrogen, -I, -Br, -Cl, and -F;
Ar₁ is selected from the group consisting of phenyl, pyridinyl, thienyl, furanyl, thiazolyl, and 1, 3, 4-thiadiazolyl; each of which is independently unsubstituted or substituted with one substituent selected form the group consisting of -I, -Br, -Cl, and -F;
Ar₂ is selected from the group consisting of thienyl, thiazolyl, isoxazolyl, 1,2,4-thiadiazolyl, and 1,2,4-oxadiazolyl; each of which is independently unsubstituted or substituted with one substituent selected from the group consisting of methyl and ethyl;
R₁₀ is selected form the group consisting of methyl and trifluoromethyl;
Z is selected form the group consisting of -OR₉ₐ and -NR₆₀R_{9b}; wherein R₉ₐ is-S(O)₂(methyl), R₆₀ is hydrogen, and R_{9b} is selected from the group consisting of hydrogen, -C(O)NH₂, -C(O)N(H)(methyl), -C(O)O(methyl), -S(O)₂(methyl), -CH₂-C(O)O(methyl) and -C(O)R₂₀ wherein R₂₀ is methyl, ethyl, isopropyl or unsubstituted cyclopropyl;
Y is -O-; and
R₅₀ is selected from the group consisting of methyl, ethyl, isopropyl, 2-methylpropyl, -R₈₀, and-CH₂-R₈₀, wherein R₈₀ at each occurrence is an unsubstituted ring selected from the group consisting of phenyl, cyclopropyl, cyclopentyl, cyclohexyl, tetrahydrofuranyl and tetrahydropyranyl,
or a pharmaceutically acceptable salt, solvate or hydrate thereof.

An embodiment of said compound is the compound:

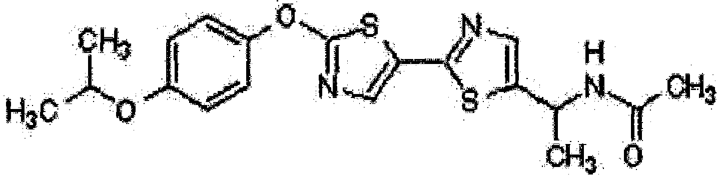

### N-(1-(2'-(4-isopropoxyphenoxy)-2,5'-bithiazol-5-yl)ethyl)acetamide

In some embodiments the compounds for use or the compound of the pharmaceutical composition for use having a structure according to Formula I, Formula la, Formula II and Formula III, as disclosed herein, are useful for the treatment of a mycobacterial disease, wherein the mycobacterial disease is caused by at least one bacteria selected from the group consisting of *Mycobacterium tuberculosis, Mycobacterium africanum, Mycobacterium bovis, Mycobacterium caprae, Mycobacterium microti, Mycobacterium leprae, Mycobacterium lepromatosis, Mycobacterium avium, Mycobacterium silvaticum, Mycobacterium hominlssuis, Mycobacterium paratuberculosis, Mycobacterium kansasii, Mycobacterium xenopi, Mycobacterium simiae, Mycobacterium abcessus, Mycobacterium fortuitum, Mycobacterium chelonae, Mycobacterium ulcerans, Mycobacterium marinum* and/or *Mycobacterium fortuitum,* preferably *Mycobacterium tuberculosis, Mycobacterium africanum, Mycobacterium bovis, Mycobacterium caprae, Mycobacterium microti, Mycobacterium leprae, Mycobacterium lepromatosis* and/or *Mycobacterium kansasii,* more preferably *Mycobacterium tuberculosis, Mycobacterium africanum, Mycobacterium bovis, Mycobacterium caprae, Mycobacterium microti* and/or *Mycobacterium kansasii,* even more preferably *Mycobacterium tuberculosis.*

Mycobacterial diseases are caused by mycobacteria. Tuberculosis and leprosy (Hansen's disease) are the best known mycobacterial diseases. However, people may also be infected by any of a group of mycobacterial species collectively called non-tuberculous mycobacteria. While tuberculosis and leprosy are most common in resource-limited countries, non-tuberculous mycobacterial infections occur worldwide.

Tuberculosis is caused by bacteria of the *Mycobacterium tuberculosis* Complex. The *Mycobacterium tuberculosis* Complex includes bacteria selected from the group consisting of *Mycobacterium tuberculosis, Mycobacterium africanum, Mycobacterium bovis, Mycobacterium caprae and Mycobacterium microfi.* In some embodiments the mycobacterial disease is caused by at least one bacteria of the *Mycobacterium tuberculosis* Complex. In some embodiments the mycobacterial disease is caused by *Mycobacterium tuberculosis.* In some embodiments the mycobacterial disease is caused by *Mycobacterium africanum.* In some embodiments the mycobacterial disease is caused by *Mycobacterium bovis.* In some embodiments the mycobacterial disease is caused by *Mycobacterium caprae.* In some embodiments the mycobacterial disease is caused by *Mycobacterium microti.* In some embodiments the mycobacterial disease is tuberculosis and/or caused by bacteria of the group consisting of *Mycobacterium tuberculosis, Mycobacterium africanum, Mycobacterium bovis, Mycobacterium caprae, Mycobacterium microti,* preferably *Mycobacterium tuberculosis.* Leprosy (also known as Hansen's disease) is caused by *Mycobacterium leprae.* In some embodiments the mycobacterial disease is caused by *Mycobacterium leprae.* The mycobacterial disease may be also caused by non-tuberculous mycobacteria (NTM). As used herein and throughout the entire description, the term "non-tuberculous mycobacteria" means mycobacteria which do not cause tuberculosis or leprosy. In children, NTM cause lymphadenitis, skin and soft tissue infections, and occasionally also lung disease and disseminated infections. Manifestations can be indistinguishable from tuberculosis on the basis of clinical and radiological findings and tuberculin skin testing. Although over 150 different species of NTM have been described, pulmonary infections are most commonly due to *Mycobacterium avium* complex (MAC), *Mycobacterium kansasii,* and *Mycobacterium abscessus.* In some embodiments the non-tuberculosis bacteria are selected from a group consisting of *Mycobacterium avium, Mycobacterium silvaticum, Mycobacterium hominissuis, Mycobacterium paratuberculosis, Mycobacterium kansasii, Mycobacterium xenopi, Mycobacterium simiae, Mycobacterium abcessus, Mycobacteriurn fortuitum, Mycobacterium chelonae, Mycobacterium ulcerans, Mycobacterium marinum, Mycobacterium gordonae* and/or *Mycobacterium fortuitum.*

The *Mycobacterium avium* complex (MAC) includes bacteria selected from the group consisting of *Mycobacterium avium, Mycobacterium silvaticum, Mycobacterium hominissuis* and *Mycobacterium paratuberculosis.* In some embodiments the mycobacterial disease is caused by non-tuberculous mycobacteria. In some embodiments the mycobacterial disease is caused by at least one bacteria of the *Mycobacterium avium* complex (MAC). In some embodiments the mycobacterial disease is caused by *Mycobacterium avium.* In some embodiments the mycobacterial disease is caused by *Mycobacterium silvaticum.* In some embodiments the mycobacterial disease is caused by *Mycobacterium hominissuis.* In some embodiments the mycobacterial disease is caused by *Mycobacterium paratuberculosis.*

In some embodiments the mycobacterial disease is caused by *Mycobacterium kansasii. Mycobacterium kansasii* causes chronic pulmonary infection that resembles pulmonary tuberculosis. However, it may also infect other organs. *M kansasii* infection is the second-most-common non-tuberculous opportunistic mycobacterial infection associated with HIV/AIDS.

In some embodiments the mycobacterial disease is caused by *Mycobacterium xenopi.* In some embodiments the mycobacterial disease is caused by *Mycobacterium simiae.* In some embodiments the mycobacterial disease is caused by *Mycobacterium abcessus.* In some embodiments the mycobacterial disease is caused by *Mycobacterium fortuitum, Mycobacterium chelonae.*

In some embodiments the mycobacterial disease is caused by *Mycobacterium ulcerans.* In some embodiments the mycobacterial disease is caused by *Mycobacterium marinum.* In some embodiments the mycobacterial disease is caused by *Mycobacterium forfuitum.* In some embodiments the mycobacterial disease is caused by *Mycobacterium gordonae.*

In some embodiments the mycobacterial disease is a pulmonary infection caused by non-tuberculous mycobacteria (NTM), preferably a chronic pulmonary infection. In some embodiments the mycobacterial disease is a skin and/or soft tissue infection caused by non-tuberculous mycobacteria (NTM). In some embodiments the mycobacterial disease is a lung disease caused by non-tuberculous mycobacteria (NTM).

In some embodiments the mycobacterial disease is selected from tuberculosis, leprosy (Hansen's disease), lepromatosis infections caused by non-tuberculosis mycobacteria including lymphadenitis and pulmonary infections, skin infections caused by mycobacteria including Buruli ulcer and fish tank granuloma. In some embodiments the mycobacterial disease is tuberculosis. In some embodiments the mycobacterial disease is leprosy (Hansen's disease). In some embodiments the mycobacterial disease is lepromatosis. In some embodiments the mycobacterial disease is a skin infection caused by mycobacteria including Buruli ulcer and fish tank granuloma. In some embodiments the mycobacterial disease is multidrug-resistant tuberculosis (MDR-TB). In some embodiments the mycobacterial disease is extensively drug-resistant tuberculosis (XDR-TB).

The compounds being inhibitors of the ACC, like the compounds of Formula I, or Formula II, and pharmaceutically-acceptable salts, solvates and hydrates thereof may be used on their own but will generally be administered in the form of a pharmaceutical composition in which the inhibitor, like the formula (I) or (II) compound/salt/solvate (active ingredient) is in association with a pharmaceutically-acceptable adjuvant, diluent or carrier.

Depending on the mode of administration, the pharmaceutical composition will preferably comprise from 0.05 to 99 % w (per cent by weight), more preferably from 0.10 to 70 % w, of active ingredient, and, from 1 to 99.95 % w, more preferably from 30 to 99.90 % w, of a pharmaceutically-acceptable adjuvant, diluent or carrier, all percentages by weight being based on total composition. The pharmaceutical composition may additionally contain an additional pharmaceutically active agent, such as an antibiotic, antifungal or anti-HIV compound and/or various other ingredients known in the art, for example, a lubricant, stabilising agent, buffering agent, emulsifying agent, viscosity regulating agent, surfactant, preservative, flavouring or colorant.

The compounds for use or the compound of the pharmaceutical composition for use being inhibitors of the ACC, like the compounds of Formula I, or Formula II, are in particular useful as bactericidal agents and/or bacteriostatic agents. The compounds are also non-hazardous for bacteria of gram-positive bacterial strains other than mycobacterial strains and non-hazardous for gram-negative bacterial strains, in particular they do not inhibit the growth of gram-negative bacterial strains and gram-positive bacterial stains other than mycobacterial strain. In some embodiments the compounds for use or the compound of the pharmaceutical composition for use being inhibitors of the ACC, like the compounds of Formula I, or Formula II, as disclosed herein, are characterized in that they are bacteriostatic, preferably bacteriostatic for mycobacteria. In some embodiments the compounds for use or the compound of the pharmaceutical composition for use being inhibitors of the ACC, like the compounds of Formula I, or Formula II, as disclosed herein, are characterized in that they are bactericidal, preferably bactericidal for mycobacteria. In some embodiments the compounds for use or the compound of the pharmaceutical composition for use being inhibitors of the ACC, like the compounds of Formula I, or Formula II, as disclosed herein, are characterized in that they are non-hazardous for bacteria of gram-positive bacterial strains other than mycobacterial strains and non-hazardous for gram-negative bacterial strains, in particular the growth of gram-negative bacterial strains and gram-positive bacterial stains other than mycobacterial strain is not inhibited. In some embodiments the compounds for use or the compound of the pharmaceutical composition for use being inhibitors of the ACC, like the compounds of Formula I, or Formula II, as disclosed herein, are characterized in that they are non-hazardous for bacteria of gram-positive bacterial strains other than mycobacterial strains, in particular the growth of gram-positive bacterial stains other than mycobacterial strain is not inhibited. In some embodiments the compounds for use or the compound of the pharmaceutical composition for use being inhibitors of the ACC, like the compounds of Formula I, or Formula II, as disclosed herein, are characterized in that they are non-hazardous for gram-negative bacterial strains, in particular the growth of gram-negative bacterial strains is not inhibited.

In some embodiments, the pharmaceutical composition for use as disclosed herein, further comprises at least one pharmaceutically acceptable carrier. In some embodiments, the compounds being inhibitors of the ACC, like the compounds of Formula I, or Formula II, or a pharmaceutically acceptable salt, solvate or hydrate thereof may be included in a pharmaceutically acceptable carrier.

As used herein and throughout the entire description, the terms "carrier" and "excipient" are used interchangeably herein. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/ro-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), anti-foaming agents (e.g. Simethicone), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc), humectants (e.g. propylene, glycol, glycerol, sorbitol). The person skilled in the art will readily be able to choose suitable pharmaceutically acceptable carriers or excipients, depending, e.g., on the formulation and administration route of the pharmaceutical composition.

A non-exhaustive list of exemplary pharmaceutically acceptable carriers or excipients includes (biodegradable) liposomes; microspheres made of the biodegradable polymer poly(D,L)-lactic-coglycolic acid (PLGA), albumin microspheres; synthetic polymers (soluble); nanofibers, protein-DNA complexes; protein conjugates; erythrocytes; or virosomes. Various carrier based dosage forms comprise solid lipid nanoparticles (SLNs), polymeric nanoparticles, ceramic nanoparticles, hydrogel nanoparticles, copolymerized peptide nanoparticles, nanocrystals and nanosuspensions, nanocrystals, nanotubes and nanowires, functionalized nanocarriers, nanospheres, nanocapsules, liposomes, lipid emulsions, lipid microtubules/microcylinders, lipid microbubbles, lipospheres, lipopolyplexes, inverse lipid micelles, dendrimers, ethosomes, multicomposite ultrathin capsules, aquasomes, pharmacosomes, colloidosomes, niosomes, discomes, proniosomes, microspheres, microemulsions and polymeric micelles. Other suitable pharmaceutically acceptable excipients are *inter alia* described in Remington's Pharmaceutical Sciences, 15th Ed., Mack Publishing Co., New Jersey (1991) and Bauer et al., Pharmazeutische Technologie, 5th Ed., Govi-Verlag Frankfurt (1997).

The pharmaceutical composition of the invention will generally be designed for specific routes and methods of administration, for specific dosages and frequencies of administration, for specific treatments of specific diseases, with ranges of bioavailability and persistence, among other things. The materials of the composition are preferably formulated in concentrations that are acceptable for the site of administration.

Formulations and compositions thus may be designed in accordance with the invention for delivery by any suitable route of administration. In the context of the present invention, the routes of administration include:
- topical routes (such as epicutaneous, inhalational, nasal, opthalmic, auricular / aural, vaginal, mucosal) and aerosols;
- enteral routes (such as oral, gastrointestinal, sublingual, sublabial, buccal, rectal); and
- parenteral routes (such as intravenous, intraarterial, intraosseous, intramuscular, intracerebral, intracerebroventricular, epidural, intrathecal, subcutaneous, intraperitoneal, extra-amniotic, intraarticular, intracardiac, intradermal, intralesional, intrauterine, intravesical, intravitreal, transdermal, intranasal, transmucosal, intrasynovial, intraluminal).

In some embodiments the administration may be a parenteral route, in particular intravenous or intramuscular.

In some embodiments, the pharmaceutical composition, as disclosed herein, is administered to a subject in need thereof in an amount effective to treat said mycobacterial disease. The subject is preferably a mammal. The subject is more preferably a human subject. The mycobacterial disease can be any mycobacterial disease disclosed herein above and below.

As used herein and throughout the entire description, the term "Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like. The subject is preferably a mammal, more preferably a human.

As used herein and throughout the entire description, the term "amount effective" in the context of a composition or dosage form for administration to a subject refers to an amount of the composition or dosage form sufficient to provide a benefit in the treatment of mycobacterial disease, to delay or minimize symptoms associated with mycobacterial infection or mycobacterial-induced disease, or to cure or ameliorate the disease or infection or cause thereof. In particular, a therapeutically effective amount means an amount sufficient to provide a therapeutic benefit *in vivo.* Used in connection with an amount of a compound of the invention, the term preferably encompasses a non-toxic amount that improves overall therapy, reduces or avoids symptoms or causes of disease, or enhances the therapeutic efficacy of or synergies with another therapeutic agent.

Amounts effective will depend, of course, on the particular subject being treated; the severity of a condition, disease or disorder; the individual patient parameters including age, physical condition, size and weight; the duration of the treatment; the nature of concurrent therapy (if any); the specific route of administration and like factors within the knowledge and expertise of the health practitioner. These factors are well known to those of ordinary skill in the art and can be addressed with no more than routine experimentation. It is generally preferred that a maximum dose be used, that is, the highest safe dose according to sound medical judgment. It will be understood by those of ordinary skill in the art, however, that a patient may insist upon a lower dose or tolerable dose for medical reasons, psychological reasons or for virtually any other reason.

In some embodiments, the pharmaceutical composition for use, as disclosed herein, is administered to a subject in need thereof in an amount effective to treat said mycobacterial disease, wherein said subject is treated with at least one additional pharmaceutically active compound, including an antibiotic, antifungal or anti-HIV compound. The additional pharmaceutically active compound is preferably an antibiotic, antifungal and/or anti-HIV compound. The additional pharmaceutically active compound is more preferably an antibiotic. The antibiotic is preferably an antituberculosis drug or an agent or compound active against *Mycobacterium tuberculosis.*

Anti-tuberculosis (TB) drugs are classified into five groups based on evidence of efficacy, potency, drug class and experience of use. In the United States rifampicin is called rifampin. First-line anti-TB drugs (Group 1) are currently recommended in a four-drug combination for the treatment of drug-susceptible TB. Second-line anti-TB drugs (Groups 2, 3 and 4) are reserved for drug-resistant TB. Third-line anti-TB drugs (Group 5) have unclear efficacy or undefined roles.

In some embodiments the additional pharmaceutically active compound is selected from the group of First-line agents consisting of Isoniazid, Rifampicin, Ethambutol, Pyrazinamide, Rifapentin and Rrifabutin.

In some embodiments the additional pharmaceutically active compound is selected from the group of Second-line agents consisting of Aminoglycosides including Kanamycin and Amikacin, Polypeptides including Capreomycin and Viomycin and Streptomycin. In some embodiments the additional pharmaceutically active compound is selected from the group of Second-line agents consisting of fluoroquinolones including Moxifloxacin, Levofloxacin, Ofloxacin and Gatifloxacin. In some embodiments the additional pharmaceutically active compound is selected from the group of a bacteriostatic Second-line agents consisting of Thioamides including Ethionamide and protionamide, Cycloserine, Terizidone, Thioacetone and p-Aminosalicylic acid.

In some embodiments the additional pharmaceutically active compound is selected from the group of Third-line agents consisting of Clofazimine, Linezolid, Amoxicillin/clavulanate, Thioacetazone, Imipenem/cilastatin, high-dose isoniazid and Clarithromycin.

In some embodiments the additional pharmaceutically active compound is selected from the group consisting of Isoniazid, Rifampicin, Ethambutol, Pyrazinamide, Rifapentin, Rifabutin, Animoglycosides including Kanamycin and/or Amikacin, Poly-petides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone, p-Aminosalicylic acid, Clofazimine, Linezolid, Amoxicillin, Clavulanate, Thioacetazone, Imipenem, Cilastatin, Clarithromycin, Delamanid and/or Bedaquiline, Q203, BTZ043 PNU-100480 (Sutelozid), SQ109, PBTZ 169, 1599, SQ609, CPZEN-45, TBI-166, PA-824/Pretomanid (more information at http://www.newtbdrugs.org/pipeline.php).

In some embodiments the additional pharmaceutically active compound is selected from the group consisting of Isoniazid, Rifampicin, Ethambutol, Pyrazinamide, Rifapentine, Rifabutin, Animoglycosides including Kanamycin and/or Amikacin, Polypetides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone and/or *p*-Aminosalicylic acid. In some embodiments the additional pharmaceutically active compound is selected from the group consisting of Isoniazid, Rifampicin, Ethambutol, Pyrazinamide, Rifapentine and/or Rifabutin. In some embodiments the additional pharmaceutically active compound is selected from the group consisting of Isoniazid, Rifampicin, Ethambutol and/or Pyrazinamide. In some embodiments the additional pharmaceutically active compound is Isoniazid. In some embodiments the additional pharmaceutically active compound is Rifampicin. In some embodiments the additional pharmaceutically active compound is Ethambutol. In some embodiments the additional pharmaceutically active compound is Pyrazinamide. In some embodiments the additional pharmaceutically active compound is Rifabutine. In some embodiments the additional pharmaceutically active compound is Delamanid. In some embodiments the additional pharmaceutically active compound is Bedaquiline.

In some embodiments the additional pharamceutically active compound is an antifungal compound. In some embodiments the antifungal compound is selected from the group consisting of Allylamines including Terbinafin and/or Naftifin, Azole-Antimycotics including Bifonazole, Butoconazole, Clotrimazole, Econazole, Fenticonazole, Fluconazole, Isoconazole, Itraconazole, Ketoconazole, Miconazole, Oxiconazole, Posaconazole, Voriconazole, Efinaconazole, Luliconazole, Sertaconazole and/or Tioconazole, Benzylamines including Butenafine, Polyenes including Amphotericin B, Nystatin and/or Pentamycine, Morpholine-Derivates including Amorolfine, Hydroxypyridone derivates including Ciclopirox, Echinocandins including Anidulafungin, Caspofungin and/or Micafungin, Pyrimidines including Flucytosine, and Thiocarbamates including Tolnaftat, Oxaboroles including Tavaborole. In some embodiments the additional pharmaceutically active compound is Methylrosaniline. In some embodiments the additional pharmaceutically active compound is Griseofulvin.

In some embodiments the additional pharmaceutically active compound is an anti-HIV compound. In some embodiments the anti-HIV compound is selected from the group consisting of Nucleoside/Nucleotide Reverse Transcriptase Inhibitors (NRTIs) including Abacavir, Atripla, Combivir, Complera, Didanosine, Emtriva, Entecavir, Epivir, Epzicom, Retrovir, Trizivir, Truvada, Videx, Videx EC, Viread, Zerit and/or Ziagen, Non-Nucleoside Reverse Transcriptase Inhibitors (NNRTIs) including Edurant, Intelence, Rescriptor, Sustiva, Viramune and/or Viramune XR, Protease Inhibitors (Pis) including Aptivus, Crixivan, Evotaz, Invirase, Kaletra, Lexiva, Norvir, Prezcobix, Prezista, Reyataz and/or Viracept, Entry/ Fusion Inhibitors including Fuzeon, Integrase Strand Transfer Inhibitors (INSTIs) including Isentress, Tivicay and/or Vitekta, Chemokine Coreceptor Antagonists (CCR5 Antagonists) including Selzentry, Pharmacokinetic Enhancer including Cytochrome P4503A (CYP3A) Inhibitors including Tybost, and Immune-Based Therapeutics including Plaquenil.

The additional pharmaceutically active compound may be provided in form of a kit of parts with the compounds according to the present invention. Further, the administration in the method as defined herein may be at the same time or may be at different time point as well as by different routes of administration. For example, the compounds according to the present invention may be adapted for inhalation while the additional active compound may be administered by the parenteral route.

Finally, the present invention relates to a method of treating subjects afflicted with a mycobacterial disease, like any of the mycobacterial disease exemplified herein. In particular, the treatment is a combinatorial treatment using the compounds according to the present invention in combination with a first line or second line tuberculosis medicament or with other suitable pharmaceutically active compounds used for the treatment of mycobacterial diseases, for example as exemplified above.

The method includes the step of administering simultaneously, separately or sequentially the compounds as defined herein with the additional compound mentioned.

The skilled person is well aware of suitable ways of administration for treating subjects afflicted with mycobacterial diseases accordingly.

### Brief description of the drawings:

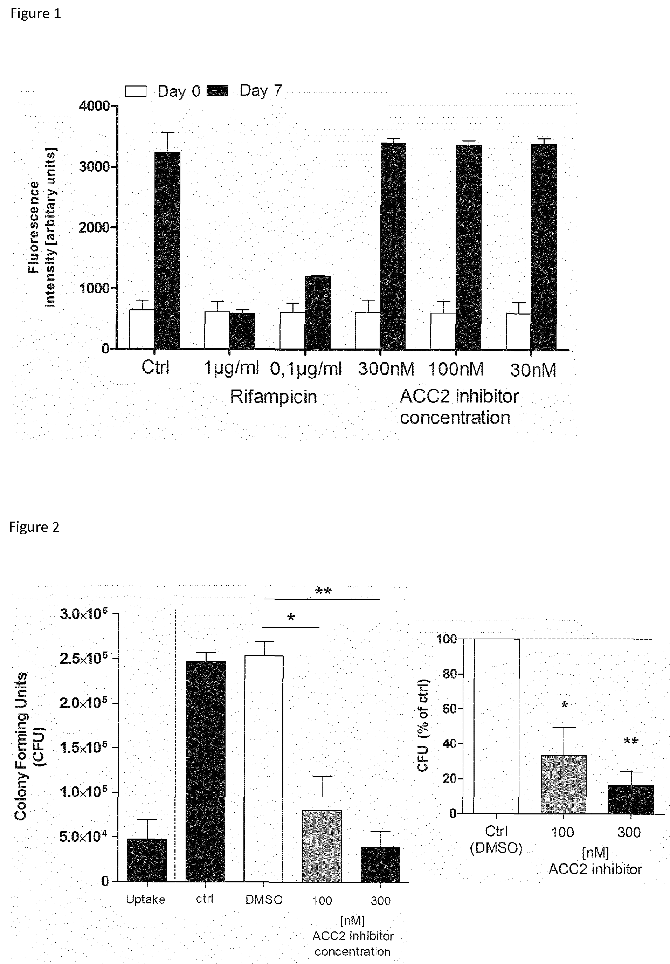

Figure 1: **Acetyl-CoA Carboxylase 2 (ACC2) inhibitor ab142090 does not affect the growth of *Mycobacterium tuberculosis* (Mtb) in liquid culture.** Green-fluorescence protein (GFP)-expressing Mtb bacteria were cultivated in liquid culture in the absence (Ctrl) or presence of the indicated concentrations of an ACC2 inhibitor (ab142090) or the antibiotic rifampicin. Growth was determined by measuring fluorescence signal intensity at 528 nm after excitation at 485 nm in a microplate reader at the indicated time points. Depicted is the mean +/- SEM of two independent experiments.
   The invention will be described by way of examples further without restricting the invention thereto.
Figure 2: **Acetyl-CoA Carboxylase 2 (ACC2) inhibitor ab142090 dose-dependently reduces intracellular growth of *Mycobacterium tuberculosis* in primary human macrophages.** Human monocyte-derived macrophages (hMDM) of healthy donors were infected with *Mycobacterium tuberculosis* (Mtb H37Rv, multiplicity of infection 1:1) for four hours. Subsequently, cells were washed and incubated in the absence (ctrl) or presence of dimethylsulfoxid (DMSO) or various concentrations (100nM, 300nM) of an ACC2 inhibitor (ab142090) for seven days. Additional culture medium-containing inhibitor was added at day three post infection. Four hours post infection (Uptake) or at day seven post infection, cells were lysed and intracellular bacteria were plated in serial dilutions on 7H10 agar plates to determine the Colony Forming Units (CFU). Data is depicted as mean +/- SEM of three independent experiments (donors) which either shows the CFU per Well (left site) or the percentage of CFU relative to DMSO-treated cells (% of ctrl, right site). For statistical analysis, raw data was log-transformed and a 1WAY-ANOVA with Bonferroni-correction as post hoc test was used to compare data from DMSO-treated macrophages with inhibitor-treated cells (*p<0.05, **p≤0.01).

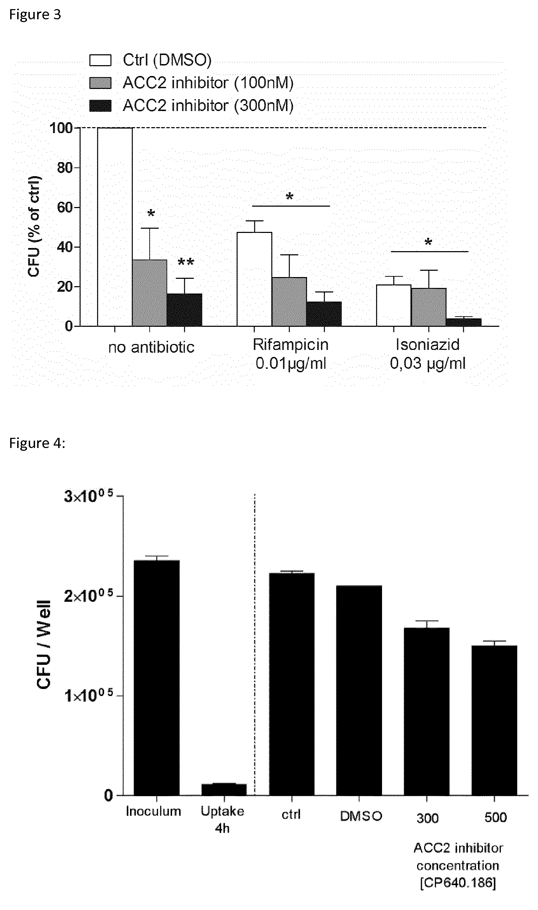

Figure 3: **Acetyl-CoA Carboxylase 2 (ACC2) inhibitor ab142090 in combination with the first line TB-drugs rifampicin and isoniazid reduces intracellular growth of *Mycobacterium tuberculosis* in primary human macrophages.** Human monocyte-derived macrophages (hMDM) of healthy donors were infected with *Mycobacterium tuberculosis* (Mtb H37Rv, multiplicity of infection 1:1) for four hours. Subsequently, cells were washed and incubated for seven days with different ACC2 inhibitor ab142090 concentrations (100nM or 300nM) in the absence (no antibiotic) or presence of the antibiotics rifampicin or isoniazid in suboptimal concentrations as indicated. Additional culture medium-containing inhibitor or antibiotic was added at day three post infection. At day seven post infection, intracellular bacteria were plated in serial dilutions on 7H10 plates to determine the Colony Forming Units (CFU). Data was normalized to DMSO-treated cells and is depicted as the mean +/- SEM of three independent experiments (donors). For statistical analysis, CFU data was log-transformed and a 1WAY-ANOVA with Bonferroni-correction as post hoc test was used to compare DMSO-treated macrophages with inhibitor treated macrophages (no antibiotic, left side) or the indicated groups treated with antibiotics (*p≤0.05).
Figure 4: **Acetyl-CoA Carboxylase 2 (ACC2) inhibitor CP640.186 dose-dependently reduces intracellular growth of *Mycobacterium tuberculosis* in primary human macrophages.** Human monocyte-derived macrophages (hMDM) of healthy donors were infected with *Mycobacterium tuberculosis* (Mtb H37Rv, multiplicity of infection 1:1) for four hours. Subsequently, cells were washed and incubated in the absence (ctrl) or presence of dimethylsulfoxid (DMSO) or various concentrations (300nM, 500nM) of an ACC2 inhibitor (CP640.186) for seven days. Additional culture medium-containing inhibitor was added at day three post infection. Four hours post infection (Uptake) or at day seven post infection, cells were lysed and intracellular bacteria were plated in serial dilutions on 7H10 agar plates to determine the Colony Forming Units (CFU). Data is depicted as mean +/- SEM of one out of two independent experiments (donors) which shows the CFU per Well.

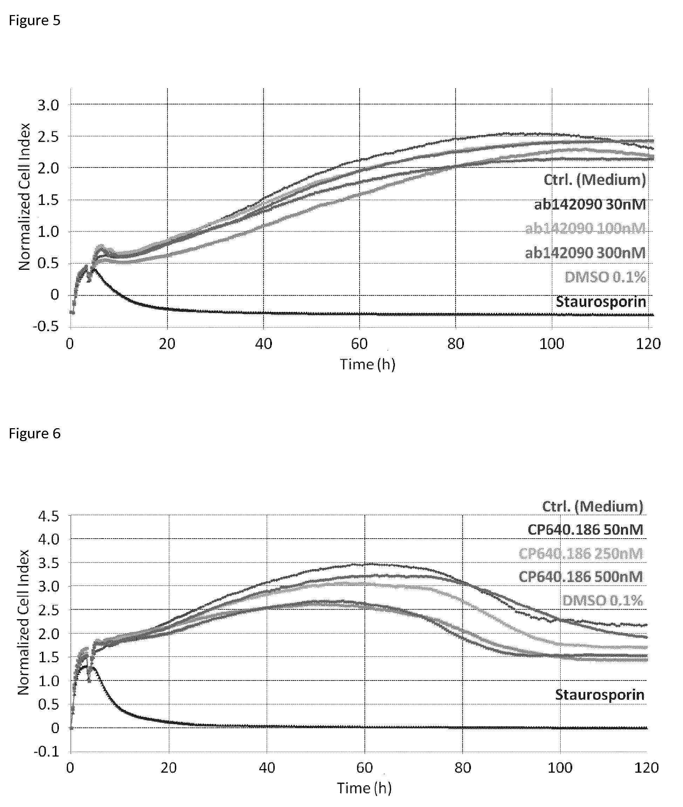

Figure 5: **Acetyl-CoA Carboxylase 2 (ACC2) inhibitor ab142090 does not affect the viability of primary human macrophages within the range of tested concentrations.** Human monocyte-derived macrophages (hMDM) were seeded in E-Plates (ACEA) and were incubated in the absence (Medium, control) or presence of DMSO (0.1%; solvent control), the apoptosis inducing agent staurosporine (1µg/ml) or the ACC2-inhibitor ab142090. The viability of macrophages is reflected by changes in the cell index, which depends on the electrical impedance on the bottom of the plate (monitored over a period of five days (120h) by use of a xCELLigence Real-Time Cell Analyzer (RTCA, ACEA)). Depicted is one representative experiment out of two.
Figure 6: **Acetyl-CoA Carboxylase 2 (ACC2) inhibitor CP640.186 does not affect the viability of primary human macrophages within the range of tested concentrations.** Human monocyte-derived macrophages (hMDM) were seeded in E-Plates (ACEA) and were incubated in the absence (Medium),or presence of DMSO (0.1 %; solvent control), the apoptosis inducing agent staurosporine (1 µg/ml) or the ACC2-Inhibitor CP640.186 at the concentrations indicated. The viability of macrophages is reflected by changes in the cell index, which depends on the electrical impedance on the bottom of the plate (monitored over a period of five days (120h) by use of a xCELLigence Real-Time Cell Analyzer (RTCA, ACEA)). Depicted is one representative experiment out of two.

### Examples

### Methods:

### 1) Measurement of Acetyl-CoA Carboxylase Inhibitory Activity and adaptation of ACC Inhibition High Throughput Screening Format using a radiochemical method: (Harwood HJ Jr, et al. (2003), J Biol Chem 278:37099-37111.)

The procedure for measuring ACC1 and ACC2 activity utilizes a radiochemical method that measures incorporation of [14C]bicarbonate into [14C]malonyl-CoA and separates product from unused substrate at the end of the reaction through acidification, which serves to both quench the reaction and remove residual radiolabeled substrate as 14CO2.

### 2) Test of ACC2 inhibition in tissue / Detection of Malonyl CoA: The activity of ACC2 inhibition leading to reduced Malonyl Co A levels in tissue samples has been described before (Glund et al. Diabetologia (2012) 55:2044-2053.

### Malonyl-CoA measurement

Animals were killed and liver and muscle samples were immediately snap frozen in liquid N2. Aliquots were homogenised in 0.5 mol/l perchloric acid and subsequently filtered using Vivaspin Centrifugal Concentrators with a 0.2 µmpolyethersulfon membrane (VWR, Darmstadt, Germany). The sample was cleaned online using a weak anion-exchange column (Oasis WAX, Waters, Eschborn, Germany) with 1 mmol/l acetic acid (pH 3.5) as loading buffer and 200 mmol/l NH3 (pH 11) for elution made up 1:9 with solvent A (5 mmol/l dibutylamine [DBA], 15 mmol/l NH4OAc pH 7) for transfer onto the reverse-phase (RP) precolumn. lonpairing RP-HPLC separation was carried out at 40°C using a precolumn and separating column Zorbax Eclipse XDB-C18 (Agilent, Böblingen, Germany), solvent A and for a gradient solvent B (5 mmol/l DBA, 15 mmol/l NH4OAc pH 7, in 50% acetonitrile). At 9.5 min, HPLC eluent was directed to an electrospray ionisation MS (Agilent 1946D) and data acquisition was started. Malonyl-CoA was detected at 854 m/z.

### 3) ACC test using a ACC/FAS coupled assay: ACC inhibition has been described by (T. S. Haque et al. / Bioorg. Med. Chem. Lett. 19 (2009) 5872-5876). The authors use a ACC/Fatty-Acid-Synthase (FAS)-Coupled Assay according to Seethala et al. (Anal.Biochem. 2006, 58, 257) with minimal modifications.

Briefly, the assay buffer (50 mM HEPES pH 7.5, 10 mM sodium citrate, 20 mM MgCl2, 6mM NaHCO3) and substrate mixture (containing 2.4 microM [3H] acetyl-CoA (PerkinElmer, NET-290) and 47.6 microM acetyl-CoA, 100 microM NADPH and 0.125 mM ATP in Assay Buffer) were all made fresh on the day of assay from stock solutions. Human ACC1 and human ACC2 were recombinant enzymes expressed in and purified from a bacculovirus system (Protein Expr. Purif. 2007, 51, 11). To each well containing 0.5 microL of compound in DMSO or DMSO as control in a 384-well phospholipid FlashPlate_ (PerkinElmer) was added 30 microL of a solution of ACC (2-4.5 nM) and FAS (1 microg/assay) enzymes in assay buffer. After a 10 min incubation, the reaction was started via addition of 20 microL of substrate mixture. The reaction was carried out for 30 min at room temperature. After incubation, the reaction was quenched with the addition of 10 microL of 200 mM EDTA (~33 mM final concentration). The [3H]-palmitic acid produced was determined by counting in a TopCount instrument (PerkinElmer). The IC50 for each compound was calculated using a logistic 4 parameter fit equation: y = A + ((B - A)/(1+((C/x)^D))) in an in house developed data processing program TOOLSET.

### 4) ACC test using purified recombinant human ACC2: Human ACC2 inhibition is measured using purified recombinant human ACC2 (hACC2) (J. W. Corbett et al. / Bioorg. Med. Chem. Lett. 20 (2010) 2383-2388) using the Transcreener ADP detection FP assay kit (Bellbrook Labs, Madison, WI) using the manufacturers' conditions for a 50 microM ATP reaction.

Human ACC2 inhibition is measured using purified recombinant human ACC2 (hACC2). A full length Cytomax clone of hACC2 was purchased from Cambridge Bioscience Limited and was sequenced and subcloned into PCDNA5 FRT TOTOPO (Invitrogen, Carlsbad, CA). The hACC2 was expressed in CHO cells by tetracycline induction and harvested in 5 L of DMEM/F12 with glutamine, biotin, hygromycin and blasticidin with 1 microg/mL tetracycline. The conditioned medium containing hACC2 was then applied to a Softlink Soft Release Avidin column (Promega, Madison, WI) and eluted with 5 mM biotin. hACC2 (4 mg) was eluted at a concentration of 0.05 mg/mL with an estimated purity of 95%. The purified hACC2 was dialyzed in 50 mM Tris, 200 mM NaCI, 4 mM DTT, 2 mM EDTA, and 5% glycerol. The pooled protein was frozen and stored at _80 _C, with no loss of activity upon thawing. For measurement of hACC2 activity and assessment of hACC2 inhibition, test compounds are dissolved in DMSO and added to the hACC2 enzyme as a 5x stock with a final DMSO concentration of 1%. rhACC2 was assayed in a Costar #3767 (Costar, Cambridge, MA) 384-well plate using the Transcreener ADP detection FP assay kit (Bellbrook Labs, Madison, WI) using the manufacturers' conditions for a 50 microM ATP reaction. The final conditions for the assay are 50 mM HEPES, pH 7.5, 5 mM MgCl2, 5 mM tripotassium citrate, 2 mM DTT, 0.5 mg/mL BSA, 30 microM acetyl-CoA, 50 microM ATP, and 8 mM KHCO3. Typically, a 10 microL reaction is run for 1 h at room temperature and 10 microL of Transcreener stop and detect buffer is added and incubated for an additional 1 h. The data is acquired on an Envision Fluorescence reader (Perkin Elmer) using a 620 excitation Cy5 FP general dual mirror, 620 excitation Cy5 FP filter, 688 emission (S) and a 688 (P) emission filter.

### 1) Mycobacterium tuberculosis Growth Analysis in Liquid Culture

GFP-expressing *Mycobacterium tuberculosis* H37Rv (Michelucci, A., et al., Proc Natl Acad Sci USA, 2013. 110(19): p. 7820-5) were generated using the plasmid 32362:pMN437 (Addgene), kindly provided by M. Niederweis (University of Alabama, Birmingham, AL) (Song, H., et al., Tuberculosis (Edinb), 2008. 88(6): p. 526-44). 1x10⁶ bacteria were cultured in 7H9 medium supplemented with oleic acid-albumin-dextrose-catalase (OADC) (10%), Tween 80 (0.05%), and glycerol (0.2%) in a total volume of 100 µl in a black 96 well plate with clear bottom (Corning Inc, Corning, NY) sealed with an air-permeable membrane (Porvair Sciences, Dunn Labortechnik, Asbach, Germany). Growth was as measured as RLU (relative light units) at 528 nm after excitation at 485 nm in a fluorescence microplate reader (Synergy 2, Biotek, Winooski, VT) at indicated time points, see figure 1.

### 2) Analysis of M. tuberculosis growth in human primary macrophages

Mononuclear cells were isolated from peripheral blood (PBMC) of healthy volunteers by density gradient centrifugation. Monocytes were separated (purity consistently >95%) by counterflow elutriation. Human monocyte-derived Macrophages (hMDM) were generated in the presence of 10 ng/ml recombinant human macrophage colony-stimulating factor (M-CSF) from highly purified monocytes as described (Reiling, N., et al., J Immunol, 2001. 167(6): p. 3339-45). *M*. *tuberculosis* growth in human macrophages was analyzed as described (Reiling, N., et al., MBio, 2013. 4(4)). In brief 2x10⁵ hMDMs were cultured in 500 µl RPMI 1640 with 10% FCS and 4 mM L-glutamine in 48-well flat-bottom microtiter plates (Nunc) at 37°C in a humidified atmosphere containing 5% CO₂. Macrophages were infected with *M. tuberculosis* strain H37Rv with a multiplicity of infection (MOI) of 1:1. Four hours post-infection, non-phagocytosed bacteria were removed by washing three times with 0.5 ml Hanks' balanced salt solution (HBSS; Invitrogen) at 37°C. After washing and after 3 days of cultivation, 0.5 ml media containing the indicated inhibitors or antibiotics was added to the macrophage culture. At day 7 supernatants were completely removed and macrophage cultures were lysed by adding Saponin (Sigma, final concentration 0.2%) in HBSS at 37°C for 15 min. Lysates were serially diluted in sterile water containing 0.05% Tween 80 (Merck, Darmstadt, Germany) and plated twice on 7H10 agar containing 0.5% glycerol (Serva) and 10% heat-inactivated bovine calf serum (BioWest, France). After 3 weeks at 37°C the colony forming units (CFUs) were counted, see figures 2, 3, and 4.

### 3) Cell Viability Analysis by Impedance Measurements

Real-time viability assays (5x10⁴ human monocyte derived macrophages/well) were performed with the xCELLigence System (Acea Biosciences Inc.) (Otero-González L et al., Environ Sci Technol. 2012; 46(18):10271-8). Impedance measurement was carried out using plates with incorporated sensor array (E-Plate) and the Real-Time Cell Analyzer (RTCA) SP instrument for 120h. After equlibration of the cells in RPMI 1640 with 10% FCS and 4mM L-glutamine for 3h, cells incubated in the absence (medium) or the presence of the ACC2 inhibitor (30, 100 and 300 nM) and staurosporine (1 µg/ml). Data obtained was analyzed using the RTCA Software 2.0 (Acea Biosciences Inc.) see figures 5 and 6.

## Claims

1. A therapeutically effective amount of an inhibitor of the acetyl-CoA-carboxylase (ACC) for use in the treatment of mycobacterial disease.

2. The therapeutically effective amount of an inhibitor of the acetyl-CoA-carboxylase (ACC) according to claim 1 being an ACC2 inhibitor.

3. The therapeutically effective amount of the inhibitor of the ACC for use in the treatment of mycobacterial disease according to claim 1 wherein said inhibitor is an inhibitor having a structure according to formula I 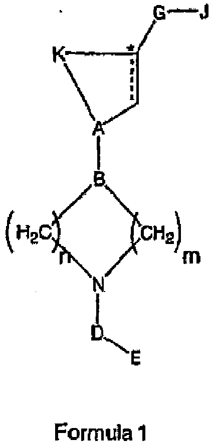 or prodrugs thereof, or pharmaceutically acceptable salts of said inhibitor or of said prodrugs;
wherein
A-B is N-CH or CH-N;
K is (CH₂)r wherein r is 2, 3 or 4;
m and n are each independently 1, 2 or 3 when A-B is N-CH or m and n are each independently 2 or 3 when A-B is CH-N;
the dashed line represents the presence of an optional double bond;
D is carbonyl or sulfonyl;
E is a tricyclic ring consisting of two fused fully unsaturated five to seven membered rings, taken independently, each of said rings optionally having one to four heteroatoms selected independently from oxygen, sulfur and nitrogen, said two fused rings fused to a third partially saturated, fully unsaturated or fully saturated five to seven membered ring, said third ring optionally having one to four heteroatoms selected independently from oxygen, sulfur and nitrogen; or
wherein said E tricyclic ring is optionally mono-, di- or tri-substituted independently on each ring used to form the tricyclic ring with halo, hydroxy, amino, cyano, nitro, oxo, carboxy, (C₁-C₆)alkyl, (C₂-C₆)alkenyl, (C₂-C₆)alkyny), (C₁-C₆)alkoxy, (C₁-C₄)alkylthio, (C₁-C₆)alkoxycarbonyl, (C₁-C₆)alkylcarbonyl, (C₁-C₆) alkylcarbonylamino, or mono-Nor di-N,N-(C₁-C₆)alkylamino, mono-N- or di-N,N-(C₁-C₆)alkylaminocarbonyl wherein said (C₁-C₆)alkyl, (C₁-C₆)alkoxy and (C₁-C₄)alkylthio substituents are also optionally mono-, di- or tri-substituted independently with chloro, bromo, hydroxy, (C₁-C₆)alkoxy, amino, mono-N- or di-N,N-(C₁-C₆)alkylamino or from one to nine fluorines; and
wherein said E tricyclic ring is optionally mono-substituted with a partially saturated, fully saturated or fully unsaturated three to eight membered ring R¹⁰ optionally having one to four heteroatoms selected independently from oxygen, sulfur and nitrogen or a bicyclic ring R¹¹ consisting of two fused partially saturated, fully saturated or fully unsaturated three to eight membered rings, taken independently, each of said rings optionally having one to four heteroatoms selected independently from oxygen, sulfur and nitrogen, said R¹⁰ and R¹¹ rings optionally additionally bridged and said R¹⁰ and R¹¹ rings optionally linked through a fully saturated, partially unsaturated or fully saturated one to four membered straight or branched carbon chain wherein the carbon(s) may optionally be replaced with one or two heteroatoms selected independently from oxygen, nitrogen and sulfur;
wherein said R¹⁰ or R¹¹ ring is optionally mono-, di or tri-substituted independently with halo, hydroxy, amino, cyano, nitro, oxo, carboxy, (C₁-C₆)alkyl, (C₂-C₆)alkenyl, (C₂-C₆)alkynyl, (C₁-C₆)alkoxy, (C₁-C₄)alkylthio, (C₁-C₆)alkoxycarbonyl, (C₁-C₆)alkylcarbonyl, (C₁-C₆)alkylcarbonylamino, or mono-N- or di-N,N-(C₁-C₆)alkylamino or mono-N- or di-N,N-(C₁-C₆)alkylaminocarbonyl wherein said (C₁-C₆)alkyl and (C₁-C₆)alkoxy substituents are also optionally mono-, di or tri-substituted independently with halo, hydroxy, (C₁-C₆)alkoxy, amino, mono-N-or diN,N-(C₁-C₆)alkylamino or from one to nine fluorines;
G is carbonyl, sulfonyl or CR⁷R⁸;
wherein R⁷ and R⁸ are each independently H, (C₁-C₆)alkyl, (C₂-C₆)alkenyl or (C₂-C₆)alkynyl or a five to seven membered partially saturated, fully saturated or fully unsaturated ring optionally having one heteroatom selected from oxygen, sulfur and nitrogen;
J is OR¹; NR²R³ or CR⁴R⁵R⁶;
wherein R¹, R² and R³ are each independently H, Q, or a (C₁-C₁₀)alkyl, (C₃-C₁₀)alkenyl or (C₃-C₁₀)alkynyl substituent wherein said carbon(s) may optionally be replaced with one or two heteroatoms selected independently from oxygen, nitrogen and sulfur and wherein said sulfur is optionally mono- or di-substituent with oxo, said carbon(s) is optionally mono-substituted with oxo, said nitrogen is optionally disubstituted with oxo, said carbon(s) is optionally mono-, di- or tri-substituted independently with halo, hydroxy, amino, nitro, cyano, carboxy, (C₁-C₄)alkylthio, (C₁-C₆)alkyloxycarbonyl, mono-N- or di-N,N-(C₁-C₆)alkylamino or mono-N- or diN,N-(C₁-C₆)alkylamino-carbonyl;
and said chain is optionally mono-substituted with Q¹;
wherein Q amd Q1 are each independently a partially saturated, fully saturated or fully unsaturated three to eight membered ring optionally having one to three heteroatoms selected independently from oxygen, sulfur and nitrogen or a bicyclic ring consisting of two fused or spirocyclic partially saturated, fully saturated or fully unsaturated three to six membered rings, taken independently, said bicyclic ring optionally having one to three heteroatoms selected independently form oxygen, sulfur and nitrogen, said mono or bicyclic ring optionally additionally bridged with (C₁-C₃)alkylene wherein said (C₁-C₃)alkylene carbons are optionally replaced with one to two heteroatoms selected independently from oxygen, sulfur and nitrogen;
wherein said Q and Q¹ ring are each independently optionally mono-, di-, tri-, or tetra-substituted independently with halo, hydroxy, amino, nitro, cyano, oxo, carboxy, (C₁-C₆)alkyl, (C₂-C₆)alkenyl, (C₂-C₆)alkynyl, (C₁-C₆) alkoxy, (C₁-C₄)alkylthio, (C₁-C₆)alkoxycarbonyl, (C₁-C₆)alkylcarbonyl, (C₁-C₆)alkylcarbonylamino, (C₁-C₆)alkyloxycarbonyl, mono-N- or di-N,N-(C₁-C₆)alkylamino, mono-N- or di-N,N-(C₁-C₆)alkylaminosulfonyl, mono-N- or diN,N-(C₁-C₆)alkylaminocarbonyl, wherein said (C₁-C₆)alkyl substituent is optionally mono-, di or tri-substituted independently with halo, hydroxy, amino, nitro, cyano, oxo, carboxy, (C₁-C₆)alkoxy, (C₁-C₄)alkylthio, (C₁-C₆)alkyloxycarbonyl or mono-N- or di-N,N-(C₁-C₆)alkylamino wherein said (C₁-C₆)alkyl substituent is also optionally substituted with from one to nine fluorines;
or wherein R² and R³ can be taken together with the nitrogen atom to which they are attached to form a partially saturated, fully saturated or fully unsaturated three to eight membered ring optionally having one to three additional heteroatoms selected independently form oxygen, sulfur and nitrogen or a bicyclic ring consisting of two fused, bridged or spirocyclic partially saturated, fully saturated or fully unsaturated three to six membered rings, taken independently, said bicyclic ring optionally having one to three additional heteroatoms selected independently from oxygen, sulfur and nitrogen or a tricyclic ring consisting of three fused, bridged or spirocyclic partially saturated, fully saturated or fully unsaturated three to six membered rings, taken independently, said tricyclic ring optionally having one to three additional heteroatoms selected independently from oxygen, sulfur and nitrogen;
wherein said NR²R³ ring is optionally mono-, di-, tri- or tetra-substituted independently with R¹⁵, halo, hydroxy, amino, nitro, cyano, oxo, carboxy, (C₁-C₆)alkyl, (C₂-C₆)alkenyl, (C₂-C₆)alkynyl, (C₁-C₆)alkoxy, (C₁-C₄)alkylthio, (C₁-C₆)alkylcarbonylamino or mono-N- or di-N,N-(C₁-C₆)alkylamino, wherein said (C₁-C₆)alkyl substituent is optionally mono-, di- or tri-substituted independently with halo, hydroxy, amino, nitro, cyano, oxo, carboxy, (C₁-C₆)alkoxy, (C₁-C₄)alkylthio, (C₁-C₆)alkyloxycarbonyl, mono-N or di-N,N-(C₁-C₆)alkylamino, said (C₁-C₆)alkyl substituent is also optionally substituted with from one to nine fluorines;
wherein R¹⁵ is carbonyl, carbamoyl, sulfonyl or sulfamoyl substituted with H, (C₁-C₆)alkyl, (C₁-C₆)alkyloxy, (C₁-C₆)alkyloxycarbonyl, (C₁-C₆)alkyloxycarbonyl(C₁-C₆)alkyl, mono-N- or di-N,N-(C₁-C₆)alkylamino,
wherein said (C₁-C₆)alkyl substituent is optionally mono-, di or tri-substituted independently with halo, hydroxy, amino, nitro, cyano, oxo, carboxy, (C₁-C₆)alkoxy, (C₁-C₄)alkylthio, (C₁-C₆)alkyloxycarbonyl, (C₁-C₆)alkylcarbonyloxy, mono-N- or di-N,N-(C₁-C₆)alkylamino or the R¹⁵ carbonyl, carbamoyl, sulfonyl or sulfamoyl linked substituent is a partially saturated, fully saturated or fully unsaturated three to eight membered ring optionally linked through (C₁-C₆)alkyl and optionally having one to three heteroatoms selected independently from oxygen, sulfur and nitrogen wherein said ring is optionally mono-, di- or tri-substituted with halo, hydroxy, amino, nitro, cyano, oxo, carboxy, (C₁-C₆)alkyl, (C₂-C₆)alkenyl, (C₂-C₆)alkynyl, (C₁-C₄)alkylthio, (C₁-C₆)alkoxy, (C₁-C₆)alkylcarbonylamino, mono-N- or di-N,N-(C₁-C₆)alkylamino; wherein said NR²R³ ring is optionally substituted with a partially saturated, fully saturated or fully unsaturated three to eight membered ring optionally having one to three heteroatoms selected independently from oxygen, sulfur and nitrogen or a bicyclic ring consisting of two fused partially saturated, fully saturated o fully unsaturated three to six membered rings, taken independently, said bicyclic ring optionally having one to three heteroatoms selected independently form oxygen, sulfur and nitrogen, said mono or bicyclic ring optionally additionally bridged said ring optionally having one to three heteroatoms selected independently from oxygen, sulfur and nitrogen, wherein said (C₁-C₆)alkyl and said ring are optionally mono-, di- or tri-substituted with halo, hydroxy, amino, nitro, cyano, oxo, carboxy, (C₂-C₆)alkenyl, (C₃-C₆)alkynyl, (C₁-C₆)alkylcarbonylamino, hydroxy, (C₁-C₆)alkoxy, (C₁-C₄)alkylthio, (C₁-C₆)alkoxy, mono-N- or di-N,N-(C₁-C₆)alkylamino;
wherein R⁴, R⁵, R⁶ are independently H, halo, hydroxy, (C₁-C₆)alkyl or R⁴ and R⁵ are taken together to form a partially saturated, fully saturated or fully unsaturated three to eight membered ring, said ring optionally having one to three heteroatoms selected independently from oxygen, sulfur and nitrogen, wherein said (C₁-C₆)alkyl and said ring are optionally mono-, di- or tri-substituted with halo, hydroxy, amino, nitro, cyano, oxo, carboxy, (C₂-C₆)alkenyl, (C₃-C₆)alkynyl, (C₁-C₆)alkylcarbonylamino, hydroxy, (C₁-C₆)alkoxy, (C₁-C₄)alkylthio, (C₁-C₆)alkoxy, mono-N- or di-N,N-(C₁-C₆)alkylamino,
or a pharmaceutically acceptable salt, solvate or hydrate thereof.

4. The therapeutically effective amount of an inhibitor of ACC2 for use in the treatment of a mycobacterial disease according to claim 1 or 2, said compound having a structure according to formula II 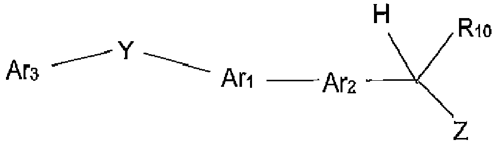
wherein R₁₀ is selected from the group consisting of hydrogen, cycloalkyl, alkyl and haloalkyl;
Y is selected form the group consisting of -(CR₄ₐR_{4b})ₚ, -C(O)-, -O-, -N(H)-, -N(alkyl)- and -S-; wherein
p is 1, 2 or 3;
each of R₄ₐ, R_{4b}, at each occurrence, is independently selected from the group consisting of hydrogen, alkyl, hydroxyalkyl, and haloalkyl when p is 1, 2 or 3; alternatively, R₄ₐ and R_{4b} together with the carbon to which they are attached form a monocyclic cycloalkyl or heterocycle ring when p is 1;
Ar₃ is 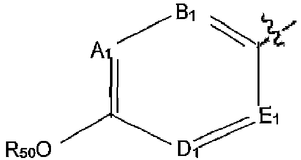
A₁, B₁, E₁, and D₁ are -C(R)-; or one of A₁, B₁, E₁ and D₁ is N and the others are-C(R)-;
wherein R is selected from the group consisting of hydrogen, -I, -Br, -Cl, and -F; An is selected from the group consisting of phenyl, pyridinyl, thienyl, furanyl, thiazolyl, and 1, 3, 4-thiadiazolyl; each of which is independently unsubstituted or substituted with one substituent selected form the group consisting of -I, -Br, -Cl, and -F;
Ar₂ is selected from the group consisting of thienyl, thiazolyl, isoxazolyl, 1,2,4-thiadiazolyl, and 1,2,4-oxadiazolyl; each of which is independently unsubstituted or substituted with one C₁-C₆ alkyl;
R₁₀ is selected form the group consisting of C₁-C₆ alkyl and haloalkyl;
Z is selected form the group consisting of -OR₉ₐ and -NR₆₀R_{9b}; wherein R₉ₐ is-S(O)₂(C₁-C₆ alkyl), R₆₀ is hydrogen, and R_{9b} is selected from the group consisting of hydrogen, -C(O)NH₂, -C(O)N(H)(C₁-C₆ alkyl), -C(O)O(C₁-C₆ alkyl), -S(O)₂(C₁-C₆ alkyl), -CH₂-C(O)O(C₁-C₆ alkyl) and -C(O)R₂₀ wherein R₂₀ is C₁-C₆ alkyl or unsubstituted C₁-C₆ cycloalkyl;
Y is -O-; and
R₅₀ is selected from the group consisting of C₁-C₆ alkyl, -R₈₀ and -(C₁-C₆ alkylenyl)-R₈₀; wherein R₈₀ at each occurrence is an unsubstituted ring selected from the group consisting of phenyl, cyclopropyl, cyclopentyl, cyclohexyl, tetrahydrofuranyl and tetrahydropyranyl,
or a pharmaceutically acceptable salt, solvate or hydrate thereof.

5. A pharmaceutical composition for use in the treatment of a mycobacterial disease wherein said composition comprises a compound as defined in any one of claims 1 to 4 optionally, with a pharmaceutically acceptable carrier, diluent or excipient.

6. The compound for use or the pharmaceutical composition for use according to any one of claims 1 to 3 and 5, wherein E is a tricyclic ring, linked through the middle ring, consisting of two fused fully unsaturated six membered rings, taken independently each of said rings optionally having a nitrogen heteroatom, said two fused rings fused to a third partially saturated or fully unsaturated six membered ring, said third ring optionally having one nitrogen heteroatom;
wherein said E ring is optionally mono-, di- or tri-substituted independently on each ring used to form the tri-cyclic or teraryl ring with halo, hydroxy, (C₁-C₆)alkyl, (C₁-C₆)alkoxy, (C₁-C₄)alkylthio, or mono-N- or diN, N-(C₁-C₆)alkylamino wherein said (C₁-C₆)alkyl and (C₁-C₆)alkoxy substituents are also optionally mono-, di- or tri-substituted independently with halo, hydroxy or from one to nine fluorines; and
J is NR²R³,
wherein R² and R³ can be taken together with the nitrogen atom to which they are attached to form a partially saturated or fully saturated five to six membered ring optionally having one additional heteroatom selected independently from oxygen and nitrogen;
wherein said NR²R³ ring is optionally mono-, di-, tri- or tetra-substituted independently with halo, hydroxy, amino, oxo, (C₁-C₆)alkyl, (C₁-C₆)alkoxy wherein said (C₁-C₆)alkyl substituent is optionally mono-, di- or tri-substituted independently with chloro, hydroxy, oxo, (C₁-C₆)alkoxy and said (C₁-C₆)alkyl substituent is also optionally substituted with from one to nine fluorines;
or wherein R² and R³ are each independently H, Q, or (C₁-C₆)alkyl,
wherein said (C₁-C₆)alkyl is optionally mono-, di- or tri-substituted independently with halo, hydroxy, (C₁-C₄)alkylthio, (C₁-C₆)alkyloxycarbonyl, or mono-N- or di-N,N-(C₁-C₆)alkylamino or Q¹;
wherein Q and Q¹ are each independently partially saturated, fully saturated or fully unsaturated three to seven membered ring optionally having one heteroatom selected independently from oxygen and nitrogen;
wherein said Q and Q¹ ring are each independently optionally mono-, di- or tri-substituted independently with halo, hydroxy, oxo, (C₁-C₆)alkyl or (C₁-C₆)alkoxy
wherein said (C₁-C₆)alkyl substituent is also optionally substituted with from one to nine fluorines or a pharmaceutically acceptable salt thereof.

7. The compound for use or the pharmaceutical composition for use according to any one of claims 1 to 3, 5 to 6 being

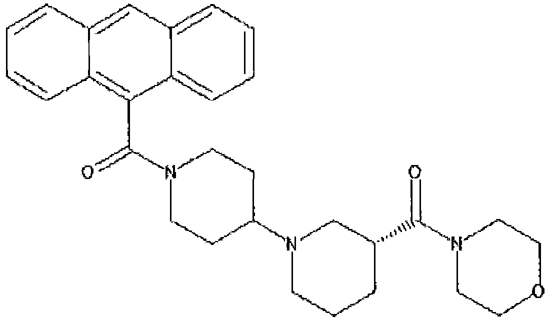

8. The compound for use or the pharmaceutical composition for use according to any one of claims 1 to 2, and 4 to 5 wherein Ar₃ is 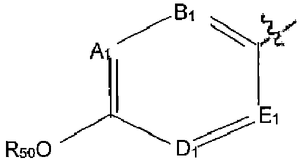
A₁, B₁, E₁, and D₁ are -C(R)-; or one of A₁, B₁, E₁ and D₁ is N and the others are-C(R)-;
wherein R is selected from the group consisting of hydrogen, -I, -Br, -Cl, and -F; An is selected from the group consisting of phenyl, pyridinyl, thienyl, furanyl, thiazolyl, and 1, 3, 4-thiadiazolyl; each of which is independently unsubstituted or substituted with one substituent selected form the group consisting of -I, -Br, -Cl, and -F;
Ar₂ is selected from the group consisting of thienyl, thiazolyl, isoxazolyl, 1,2,4-thiadiazolyl, and 1,2,4-oxadiazolyl; each of which is independently unsubstituted or substituted with one substituent selected from the group consisting of methyl and ethyl;
R₁₀ is selected form the group consisting of methyl and trifluoromethyl;
Z is selected form the group consisting of -OR₉ₐ and -NR₆₀R_{9b}; wherein R₉ₐ is-S(O)₂(methyl), R₆₀ is hydrogen, and R_{9b} is selected from the group consisting of hydrogen, -C(O)NH₂, -C(O)N(H)(methyl), -C(O)O(methyl), -S(O)₂(methyl), -CH₂-C(O)O(methyl) and -C(O)R₂₀ wherein R₂₀ is methyl, ethyl, isopropyl or unsubstituted cyclopropyl;
Y is -O-; and
R₅₀ is selected from the group consisting of methyl, ethyl, isopropyl, 2-methylpropyl, - R₈₀, and -CH₂-R₈₀; wherein R₈₀ at each occurrence is an unsubstituted ring selected from the group consisting of phenyl, cyclopropyl, cyclopentyl, cyclohexyl, tetrahydrofuranyl and tetrahydropyranyl,
or a pharmaceutically acceptable salt, solvate or hydrate thereof.

9. The compound for use or the pharmaceutical composition for use according to any one of claims 1 to 2, and 4 to 6 being

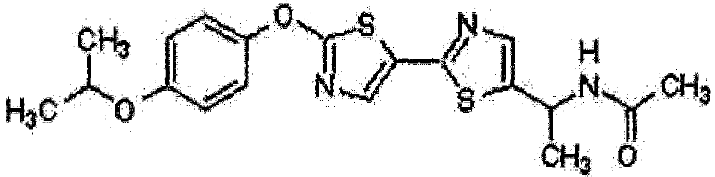

10. The compound for use or the pharmaceutical composition for use according to any one of claims 1 to 9 wherein the mycobacterial disease is caused by at least one bacteria selected from the group consisting of *Mycobacterium tuberculosis, Mycobacterium africanum, Mycobacterium bovis, Mycobacterium caprae, Mycobacterium microti, Mycobacterium leprae, Mycobacterium lepromatosis, Mycobacterium avium, Mycobacterium silvaticum, Mycobacterium hominissuis, Mycobacterium paratuberculosis, Mycobacterium kansasii, Mycobacterium xenopi, Mycobacterium simiae, Mycobacterium abcessus, Mycobacterium fortuitum, Mycobacterium chelonae, Mycobacterium ulcerans, Mycobacterium marinum* and/or *Mycobacterium fortuitum,* preferably *Mycobacterium tuberculosis, Mycobacterium africanum, Mycobacterium bovis, Mycobacterium caprae, Mycobacterium microti, Mycobacterium leprae, Mycobacterium lepromatosis* and/or *Mycobacterium kansasii,* more preferably *Mycobacterium tuberculosis, Mycobacterium africanum, Mycobacterium bovis, Mycobacterium caprae, Mycobacterium microti* and/or *Mycobacterium kansasii,* even more preferably *Mycobacterium tuberculosis,* in particular, wherein the mycobacterial disease is selected from tuberculosis and/or caused by bacteria of the group consisting of Mycobacterium *tuberculosis, Mycobacterium africanum, Mycobacterium bovis, Mycobacterium caprae, Mycobacterium microti, preferably Mycobacterium tuberculosis.*

11. The compound for use or the pharmaceutical composition for use according to any one of claims 1 to 10 wherein the mycobacterial disease is selected from tuberculosis, leprosy (Hansen's disease), lepromatosis, infections caused by non-tuberculosis mycobacteria including lymphadenitis and pulmonary infections, skin infections caused by mycobacteria including Buruli ulcer and fish tank granuloma.

12. The pharmaceutical composition for use according to any one of claims 5 to 11, wherein said composition is administered to a subject in need thereof in an amount effective to treat said mycobacterial disease preferably wherein the subject is a mammal, more preferably, a human subject.

13. The pharmaceutical composition for use according to any one of claims 5 to 12 wherein said subject is treated with at least one additional pharmaceutically active compound, including an antibiotic, antifungal or anti-HIV compound, preferably wherein said additional pharmaceutically active compound is an antibiotic, antifungal and/or anti-HIV compound, more preferably an antibiotic.

14. The pharmaceutical composition for use according to claim 13, wherein said additional pharmaceutically active compound is selected from the group consisting of Isoniazid, Rifampicin, Ethambutol, Pyrazinamide, Rifapentine, Rifabutin, Animoglycosides including Kanamycin and/or Amikacin, Polypetides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone, *p*-Aminosalicylic acid, Clofazimine, Linezolid, Amoxicillin, Clavulanate, Thioacetazone, Imipenem, Cilastatin, Clarithromycin, Delamanid and/or Bedaquiline, preferably Isoniazid, Rifampicin, Ethambutol, Pyrazinamide, Rifapentine, Rifabutin, Animoglycosides including Kanamycin and/or Amikacin, Polypetides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone and/or *p*-Aminosalicylic acid, more preferably Isoniazid, Rifampicin, Ethambutol, Pyrazinamide, Rifapentine and/or Rifabutin, even more preferably Isoniazid, Rifampicin, Ethambutol and/or Pyrazinamide.

15. Kit of parts comprising a pharmaceutical composition and at least one additional pharmaceutically active compound wherein said pharmaceutical composition comprises a compound as defined in any one of claims 1 to 4 and 5 to 9 and wherein said additional pharmaceutically active compound is selected from the group consisting of an antibiotic, antifungal or anti-HIV compound, preferably an antibiotic selected from the group of Isoniazid, Rifampicin, Ethambutol, Pyrazinamide, Rifapentin, Rifabutin, Animoglycosides including Kanamycin and/or Amikacin, Polypetides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone, *p-*Aminosalicylic acid, Clofazimine, Linezolid, Amoxicillin, Clavulanate, Thioacetazone, Imipenem, Cilastatin, Clarithromycin, Delamanid and/or Bedaquiline, preferably Isoniazid, Rifampicin, Ethambutol, Pyrazinamide, Rifapentin, Rifabutin, Animoglycosides including Kanamycin and/or Amikacin, Polypetides including Capreomycin, Viomycin and/or Streptomycin, fluoroquinolones including Moxifloxacin, Levofloxacin, Ofloxacin and/or Gatifloxacin, thioamides including Ethionamide and/or Protionamide, Cycloserine, Terizidone, Thioacetone and/or *p*-Aminosalicylic acid, more preferably Isoniazid, Rifampicin, Ethambutol, Pyrazinamide, Rifapentin and/or Rifabutin, even more preferably Isoniazid, Rifampicin, Ethambutol and/or Pyrazinamide.
